# EUROPEAN PATENT SPECIFICATION

(11) **EP 1 832 657 B1**
(45) Date of publication and mention of the grant of the patent: **24.10.2012**
(21) Application number: 06021432.7
(22) Date of filing: 09.09.1999
(51) Int. Cl.: C12N 15/85

(54) **Methylation of plasmid vectors**
Methylierung von Plasmid-Vektoren
Méthylation de vecteurs plasmidiques

(30) Priority: 09.09.1998 US 99583 P
(43) Date of publication of application: 12.09.2007
(62) Divisional of application: 99946789.7
(73) Proprietor: GENZYME CORPORATION, Cambridge, MA 02142 (US)
(72) Inventor: Cheng, Seng H., Wellesley, MA 02481 (US); Marshall, John, Hopedale, MA 01747 (US); Scheule, Ronald K., Hopkinton, MA 01748 (US); Yew, Nelson S., West Upton, MA 01568 (US); Tousignant, Jennifer D., Westford, MA 01886 (US); Przybylska, Malgorzata, Arlington, MA 02174 (US)
(74) Representative: Adams, Harvey Vaughan John

(56) References cited:
- WO-A-96/02555
- WO-A-96/26179
- WO-A-98/11211
- US-A- 5 747 471
- GILBERT W: "pUC13c cloning vector (beta-galactosidase mRNA on complementary strand)" EMBL SEQUENCE DATABASE, 25 March 1993 (1993-03-25), XP002131903 HEIDELBERG DE
- OKA ET AL.: "Nucleotide sequence of the kanamycin resistance transposon Tn903" EMBL SEQUENCE DATABASE, 9 June 1982 (1982-06-09), XP002131904 HEIDELBERG DE
- KRIEG A M ET AL: "CPG MOTIFS IN BACTERIAL DNA TRIGGER DIRECT B-CELL ACTIVATION" NATURE,GB,MACMILLAN JOURNALS LTD. LONDON, vol. 374, 6 April 1995 (1995-04-06), page 546-549, XP002910391 ISSN: 0028-0836
- DATABASE WPI Section Ch, Week 199439 Derwent Publications Ltd., London, GB; Class B04, AN 1994-312814 XP002131905 & JP 06 237773 A (SHIDA H) 30 August 1994 (1994-08-30)
- PATENT ABSTRACTS OF JAPAN vol. 1997, no. 12, 25 December 1997 (1997-12-25) & JP 09 206077 A (CALPIS FOOD IND CO LTD:THE), 12 August 1997 (1997-08-12)
- DATABASE WPI Section Ch, Week 199534 Derwent Publications Ltd., London, GB; Class D15, AN 1995-260052 XP002131906 & JP 07 163377 A (KOKUZEI CHO CHOKAN) 27 June 1995 (1995-06-27)
- PATENT ABSTRACTS OF JAPAN vol. 015, no. 259 (C-0846), 2 July 1991 (1991-07-02) & JP 03 087173 A (TEIJIN LTD;OTHERS: 01), 11 April 1991 (1991-04-11)
- PATENT ABSTRACTS OF JAPAN vol. 1998, no. 10, 31 August 1998 (1998-08-31) & JP 10 127287 A (CENTEON PHARMA GMBH), 19 May 1998 (1998-05-19)

## Description

### Technical Field

The present invention relates to a novel approach to reducing the toxicity and increasing the efficacy of gene delivery. The present invention also relates to an approach to modulating the immunostimulatory response to gene therapy, in particular the reduction of immunostimulatory responses such as inflammatory responses and reduction of stress on the liver.

The effective introduction of foreign genes and other biologically active molecules into targeted mammalian cells is a challenge still facing those skilled in the art. Gene therapy requires successful transfection of target cells in a patient. Transfection, which is practically useful per se, may generally be defined as a process of introducing an expressible polynucleotide (for example a gene, a cDNA, or an mRNA) for sense or antisense expression into a cell. Successful expression of the encoding polynucleotide thus transfected leads to production in the cells of a transcription and/or translation product and is also practically useful per se. A goal, of course, is to obtain expression sufficient to lead to correction of the disease state associated with the abnormal gene.

### Background Art

Examples of diseases that are targets of gene therapy include: inherited disorders such as cystic fibrosis, hemophilia, Gaucher's disease, Fabry's disease, and muscular dystrophy. Representative of acquired target disorders are: (1) for cancers-multiple myeloma, leukemias, melanomas, ovarian carcinoma and small cell lung cancer; (2) for cardiovascular conditions-progressive heart failure, restenosis, and hemophilias; and (3) for neurological conditions-traumatic brain injury.

Cystic fibrosis, a common lethal genetic disorder, is a particular example of a disease that is a target for gene therapy. The disease is caused by the presence of one or more mutations in the gene that encodes a protein known as cystic fibrosis transmembrane conductance regulator ("CFTR"). Cystic fibrosis is characterized by chronic sputum production, recurrent infections and lung destruction (Boat, T.F., *McGraw-Hill, Inc.,* 1989, p. 2649-2680). Though it is not precisely known how the mutation of the CFTR gene leads to the clinical manifestation (Welsh, M.J. et al. Cell 73:1251-1254, 1993), defective Cl⁻ secretion and increased Na⁺ absorption (Welsh, M.J. et al., Cell 73:1251-1254, 1993; Quinton, P.M., FASEB Lett. 4:2709-2717,1990) are well documented. Furthermore, these changes in ion transport produce alterations in fluid transport across surface and gland epithelia (Jiang, C. et al., Science 262:424-427, 1993; Jiang, C. et al., J. Physiol. (London), 501.3:637-647, 1997; Smith, J.J. et al. J. Clin. Invest., 91:1148-1153, 1993; and Zhang, Y. et al., Am.J.Physiol 270:C1326-1335, 1996). The resultant alterations in water and salt content of airway liquid (ASL) may diminish the activity of bactericidal peptides secreted from the epithelial cells (Smith, J.J. et al., Cell, 85:229-236, 1996) and/or impair mucociliary clearance, thereby promoting recurrent lung infection and inflammation.

It is widely expected that gene therapy will provide a long lasting and predictable form of therapy for certain disease states such as CF, however, there is still a need to develop improved methods that facilitate entry of functional genes into cells, and whose activity in this regard is sufficient to provide for *in vivo* delivery of genes or other such biologically active molecules.

Effective introduction of many types of biologically active molecules has been difficult and not all the methods that have been developed are able to effectuate efficient delivery of adequate amounts of the desired molecules into the targeted cells. The complex structure, behavior, and environment presented by an intact tissue that is targeted for intracellular delivery of biologically active molecules often interfere substantially with such delivery. Numerous methods and delivery vehicles including viral vectors, DNA encapsulated in liposomes, lipid delivery vehicles, and naked DNA have been employed to effectuate the delivery of DNA into the cells of mammals. To date, delivery of DNA in vitro, ex vivo, and in vivo has been demonstrated using many of the aforementioned methods.

Viral transfection, for example, has proven to be relatively efficient. However, the host immune response posseses possible problems. Specifically, viral proteins activate cytotoxicity T lymphocytes (CTLs) which destroy the virus-infected cells thereby terminating gene expression in the lungs of *in vivo* models examined. The other problem is diminished gene transfer upon repeated administration of viral vectors due to the development of antiviral neutralizing antibodies. These issues are presently being addressed by modifying both the vectors and the host immune system. Additionally, non-viral and non-proteinaceous vectors have been gaining attention as alternative approaches.

Because compounds designed to facilitate intracellular delivery of biologically active molecules must interact with both non-polar and polar environments (in or on, for example, the plasma membrane, tissue fluids, compartments within the cell, and the biologically active molecule itself), such compounds are designed typically to contain both polar and non-polar domains. Compounds having both such domains may be termed amphiphiles, and many lipids and synthetic lipids that have been disclosed for use in facilitating such intracellular delivery (whether for *in vitro* or *in vivo* application) meet this definition. One group of amphiphilic compounds that have showed particular promise for efficient delivery of biologically active molecules are cationic amphiphiles. Cationic amphiphiles have polar groups that are capable of being positively charged at or around physiological pH, and this property is understood in the art to be important in defining how the amphiphiles interact with the many types of biologically active molecules including, for example, negatively charged polynucleotides such as DNA.

Examples of cationic amphiphilic compounds that are stated to be useful in the intracellular delivery of biologically active molecules are found, for example, in the following references, the disclosures of which are specifically incorporated by reference. Many of these references also contain useful discussions of the properties of cationic amphiphiles that are understood in the art as making them suitable for such applications, and the nature of structures, as understood in the art, that are formed by complexing of such amphiphiles with therapeutic molecules intended for intracellular delivery.
(1) Feigner, et al., Proc. Natl. Acad. Sci. USA, 84, 7413-7417 (1987) disclose use of positively-charged synthetic cationic lipids including N-[1(2,3-dioleyloxy)propyl]-N,N,N-trimethylammonium chloride ("DOTMA"), to form lipid/DNA complexes suitable for transfections. See also Feigner et al., The Journal of Biological Chemistry, 269(4), 2550-2561 (1994).
(2) Behr et al., Proc. Natl. Acad. Sci., USA 86, 6982-6986 (1989) disclose numerous amphiphiles including dioctadecylamidologlycylspermine ("DOGS").
(3) U.S. Patent 5,283,185 to Epand et al. describe additional classes and species of amphiphiles including 3β [N-(N¹,N¹- dimethylaminoethane)-carbamoyl] cholesterol, termed "DC-chol".
(4) Additional compounds that facilitate transport of biologically active molecules into cells are disclosed in U.S. Patent No. 5,264,618 to Felgner et al. See also Felgner et al., The Journal of Biological Chemistry 269(4), pp. 2550-2561 (1994) for disclosure therein of further compounds including "DMRIE" 1,2-dimyristyloxypropyl-3-dimethyl-hydroxyethyl ammonium bromide, which is discussed below.
(5) Reference to amphiphiles suitable for intracellular delivery of biologically active molecules is also found in U.S. Patent No. 5,334,761 to Gebeyehu et al., and in Felgner et al., Methods (Methods in Enzymology), 5, 67-75 (1993).
(6) Brigham, K.L., B. Meyrick, B. Christman, M. Magnuson, G. King and L.C. Berry. In vivo transfection of murine lungs with functioning prokaryotic gene using a liposome vehicle Am.J.Med.Sci. 298:278-281, 1989.
(7) Gao, X.A. and L. Huang. A novel cationic liposome reagent for efficient transfection of mammalian cells. Biochem Biophys Res Commun 179:280-285, 1991.
(8) Yoshimura, K., M.A. Rosenfeld, H. Nakamura, E.M. Scherer, A. Pavirani, J.P. Lecocq and R.G. Crystal. Expression of the human cystic fibrosis transmembrane conductance regulator gene in the mouse lung after in vivo intratracheal plasmid-mediated gene transfer. Nucl.Acids Res. 20:3233-3240, 1992.
(9) Zhu, N., D. Liggitt, Y. Liu and R. Debs. Systemic gene expression after intravenous DNA delivery into adult mice. Science 261:209-211, 1993.
(10) Solodin, I., C.S. Brown, M.S. Bruno, C.Y. Chow, E. Jang, R.J. Debs and T.D. Heath. A novel series of amphiphilic imidazolinium compounds for in vitro and in vivo gene delivery. Biochem. 34:13537-13544, 1995. (11) Lee, E.R., J. Marshall, C.S. Siegal, C. Jiang, N.S. Yew, M.R. Nichols, J.B. Nietupski, R.J. Ziegler, M. Lane, K.X. Wang, N.C. Wan, R.K. Scheule, D.J. Harris, A.E. Smith and S.H. Cheng. Detailed analysis of structure and formulations of cationic lipids for efficient gene transfer to the lung. Hum.Gene Ther. 7:1701-1717, 1996.

Additionally, several recently issued U.S. Patents, the disclosures of which are specifically incorporated by reference herein, have described the utility of cationic amphiphiles to deliver polynucleotides to mammalian cells. (U.S. Patent No. 5,676,954 to Brigham et al. and U.S. Patent No. 5,703,055 to Felgner et al.)

Another class of cationic amphiphiles with enhanced activity is described, for example, in U.S. Patent No. 5,747,471 to Siegel et al. issued May 5, 1998, U.S. Patent No. 5,650,096 to Harris et al. issued July 22, 1997, and PCT publication WO 98/02191 published January 22, 1998, the disclosures of which are specifically incorporated by reference herein. These patents also disclose formulations of cationic amphiphiles of relevance to the practice of the present invention.

In addition to achieving effective introduction of biologically active molecules, there remains a need to reduce the toxicity of gene delivery. In particular, there is a need to reduce the inflammatory response associated with gene delivery. For example, cationic lipid-mediated gene transfer to the lung induces dose-dependent pulmonary inflammation characterized by an influx of leukocytes (predominantly neutrophils) and elevated levels of the inflammatory cytokines interleukin-6 (IL-6), tumor necrosis factor α (TNF-α), and interferon-γ (TNF-γ) in the bronchoalveolar lavage fluid.

The generation of elevated levels of cytokines in the BALF also has consequences for expression of the therapeutic protein. Several viral promoters such as the CMV promoter commonly used in gene delivery vectors are subject to suppression by such cytokines. Furthermore, any additional inflammation or reduction in lung function in patients that already exhibit chronically inflamed, compromised airways represents an increased safety risk. For CF and other inherited genetic disorders, a consequence of the presence of CpG motifs maybe the increased likelihood of developing neutralizing antibodies to the therapeutic transgene. This is particularly pertinent in subjects harboring either null mutations or mutations that result in the generation of a very altered variant. Eliminating the adjuvant effect of the immunostimulatory CpG motifs would be desirable to reduce this risk.

Histopathological analysis of lung sections treated with the individual components of cationic Iipid:DNA complexes suggests that the cationic lipid was a mediator of the observed inflammation. However, results of clinical studies where CF subjects were subjected to either aerosolized liposomes alone or cationic lipid:DNA complexes indicated that bacterial derived plasmid DNA may also be inflammatory. Each of the cationic lipid:pDNA-treated patients exhibited mild flu-like symptoms (including fever, myalgia, and a reduction in FEV, of approximately 15%) over a period of approximately 24 h. These symptoms were not observed in patients treated with the liposome control. One possible explanation for this response is related to the presence of unmethylated CpG dinucleotide sequences in bacterially-derived pDNA. See Krieg et al., Nature, 374, 546-9 (1995); Klinman et al., Proc. Natl. Acad. Sci. USA, 83, 2879-2883 (1996); Sato et al., Science, 273, 352-4 (1996). Unmethylated CpG dinucleotides have been shown to be immunostimulatory and are present at a much higher frequency in bacterially-derived plasmid DNA compared to vertebrate DNA.

Since plasmid DNA used in gene transfer studies are invariably isolated from bacterial sources, and because they also necessarily harbor bacterial sequences for propagation in this host, they contain a higher frequency of unmethylated CpG sequences. The presence of such motifs on pDNA have been shown to be capable of stimulating a robust T-helper 1 type response in either transfected monocytes or injected BALB/c mice. However, of particular concern for delivery of genes to the lung was the demonstration that bacterial genomic DNA or oligonucleotides containing immunostimulatory CpG motifs are capable of eliciting an acute inflammatory response in airways and in particular caused inflammation in the lower respiratory tract, increasing both cell numbers and elevated levels of the cytokines TNF-α, IL-6 and macrophage inflammatory protein (MIP-2). See Schwartz et al., J. Clin. Invest., 100, 68-73 (1997). Activation of a similar cytokine profile by CpG dinucleotides has also been reported in lymphocytes (Klinman et al., Proc. Natl. Acad. Sci. USA, 83, 2879-2883, 1996), murine dendritic cells (Sparwasser et al., Eur. J. Immunol., 28, 2045-2054, 1998), macrophages (Lipford et al., Eur. J. Immunol. 27, 2340-2344, 1997), monocytes (Sato et al., Science, 273, 352-4, 1995), and NK cells (Cowdery et al., J. Immunol., 156, 4370-4575, 1996). A recent study also reported that complexes formed between the cationic lipid DOTMA (N-[1-(2-3--dioleyloxy)propyl]-N,N,N-trimethylammonium chloride) and pDNA enhanced cytokine and cellular levels in the BALF of treated animals. See Friemark et al., J. Immunol., 160, 4580-6 (1998).

Compared to DNA of eukaryotic origin, bacterial genomic DNA contain a 20 fold higher frequency of the dinucleotide sequence CpG. Additionally, unlike eukaryotic DNA where 80% of the cytosines are methylated, those derived from prokaryotic origin are relatively unmethylated. These differences purportedly allow the vertebrate immune system to recognize and respond to foreign DNA of bacterial origin. In this regard, administration of genomic bacterial DNA into an eukaryotic host has been shown to be capable of eliciting a potent immunostimulatory response, activating B cells, NK cells, dendritic cells and macrophages. See Krieg et al., Trends Microbiol., 4, 73-76 (1995); Ballas et al., J. Immunol., 157, 1840-5 (1996); Sparwasser et.al., Eur. J. Immunol., 27, 1671-9 (1997).

Systematic analysis indicated that those sequences harboring the CpG motif 5'-RRCGYY-3' were particularly potent at inducing these responses. That these effects were a consequence of the methylation status of the CpG dinucleotide sequences were demonstrated by experiments showing that administration of either bacterial genomic DNA or synthetic oligonucleotides bearing the RRCGYY sequence that had been pre-methylated with CpG methylase were significantly less immunostimulatory.

### Disclosure of the Invention

The present disclosure provides for methods of reducing the inflammatory response to gene therapy by modifying the plasmid delivered to the cell. The plasmid is modified to reduce or eliminate the immunostimulatory response in order to preserve the efficacy of nucleic acid transfer but reduce the associated toxicity. The present disclosure provides for the modification of any plasmid for delivery to a mammalian cell. The plasmid may be an RNA plasmid or a DNA plasmid.

In one aspect, the present disclosure provides for a method of reducing an inflammatory or immunostimulatory response to gene delivery by partially or completely methylating the plasmid vector to an extent sufficient to reduce the inflammatory or immunostimulatory response. Preferably, the inflammatory response is reduced. Unmethylated plasmid DNA vectors are a major contributor to the inflammatory response associated with gene delivery. Methylation of the plasmid DNA vector reduces the inflammatory response and thus reduces the toxicity of gene therapy.

The present disclosure also provides for a method of reducing a mammal's immunostimulatory response to a composition comprising the step of administering a composition that comprises at least one plasmid that is a CpG altered plasmid and at least one cationic amphiphile. The method of altering the plasmid is chosen from removing at least one CpG motif from the plasmid,
methylating at least one CpG motif of the plasmid, or removing at least one CpG motif and methylating at least one CpG motif. The plasmid may be a DNA plasmid and also may comprise at least one modified KAN fragment, at least one modified ORI fragment or at least one modified CAT fragment. Additionally, the cationic amphiphile may be a cationic lipid

In a preferred aspect, the DNA plasmid encodes a gene of interest. The gene of interest may be but is not limited to alpha-galactosidase, Factor VIII, Factor IX, or CF. Similar to the plasmid, the gene of interest may also be CpG altered.

Another aspect is a method of reducing a mammal's immunostimulatory response to a composition comprising the altering of a plasmid by removing at least one CpG motif from the plasmid and measuring the immunostimulatory response by monitoring immunostimulated liver enzyme levels in the blood of the mammal. The immunostimulated liver enzyme levels are preferably serum transaminase factors, such as AST and/or ALT levels.

The present disclosure also provides for a method of reducing a mammal's immunostimulatory response to a composition comprising altering a plasmid by methylating at least one CpG motif of the plasmid and measuring the immunostimulatory response by monitoring the cytokine levels in the mammal.

The methods described within of reducing a mammal's immunostimulatory response to a composition may also include the administration of an agent effective to inhibit CpG signaling. The agent effective to inhibit CpG signaling may be but is not limited to monensin, bafilomycin, chloroquine, and quinacrine.

In a further aspect, the present disclosure calls for a method of modulating a mammal's immunostimulatory response to a cationic amphiphile/plasmid composition comprising modifying an amount of CpG motifs in the plasmid effective to alter the liver enzyme levels in the blood of a mammal. The CpG motifs may be modified by the removal of at least one CpG motif and/or the methylation of at least one CPG motif.

In an even further aspect, the present disclosure calls for a method of modulating a mammal's immunostimulatory response to a cationic amphiphile/plasmid composition comprising modifying an amount of CpG motifs in the plasmid effective to alter the cytokine levels in the blood of a mammal. The CpG motifs may be modified by the methylation of at least one CPG motif.

Also within the practice of the present disclosure is a composition comprising at least one CpG altered plasmid and at least one cationic amphiphile. The CpG altered plasmid differs from its corresponding wild type sequence by: the absence of at least one CpG motif from the plasmid; the presence of at least one methylated CpG in the plasmid; or the absence of at least one CpG motif from the plasmid and the presence of at least one methylated CpG in at least one CpG motif. In one aspect, the plasmid is a DNA plasmid comprising a modified CpG region having at least one CpG-reduced selectable marker, such as a CpG-deleted KAN fragment or a CpG-reduced CAT fragment, or a CpG reduced origin of replication, such as a shortened ORI region. The composition may further comprise an agent that is effective to inhibit CpG signaling.

The present disclosure also encompasses a composition comprising a polynucleotide comprising the nucleotide sequence of SEQ ID NO:1 and/or fragments of the nucleotide sequence of SEQ ID NO:1. The composition may further comprise a cationic amphiphile.

The present disclosure provides for direct administration of modified plasmid DNA, administration of plasmid DNA:lipid complexes, along with the use of modified plasmid DNA with viral vectors including adenoviruses and any other methods that have been employed in the art to effectuate delivery of biologically active molecules into the cells of mammals. In a preferred aspect, a methylated plasmid DNA vector is administered as a lipid:methylated DNA complex.

In another aspect, the present disclosure provides for pharmaceutical compositions of modified plasmid DNA complexes comprising modified plasmid DNA and pharmaceutical compositions of lipid and non-lipid complexes with modified plasmid DNA. The modified plasmid DNA may be an active ingredient in a pharmaceutical composition that includes carriers, fillers, extenders, dispersants, creams, gels, solutions and other excipients that are common in the pharmaceutical formulatory arts.

The present disclosure provides for a method of administering the modified plasmid DNA or modified plasmid DNA complex by any methods that have been employed in the art to effectuate delivery of biologically active molecules into the cells of mammals including but not limited to administration of an aerosolized solution, intravenous injection, orally, parenterally, topically, or transmucosally.

The present disclosure also provides for a pharmaceutical composition that comprises one or more lipids or other carriers that have been employed in the art to effectuate delivery of biologically active molecules into the cells of mammals, and one or more biologically active molecules or modified plasmid DNA vectors, wherein said compositions facilitate intracellular delivery in the tissues of patients of therapeutically effective amounts of the biologically active molecules or modified plasmid DNA vectors. The pharmaceutical compositions of the present disclosure may be formulated to contain one or more additional physiologically acceptable substances that stabilize the compositions for storage and/or contribute to the successful intracellular delivery of the biologically active molecules and modified plasmid DNA.

For pharmaceutical use, the cationic amphiphile(s):modified plasmid DNA complexes of the present disclosure may be formulated with one or more additional cationic amphiphiles including those known in the art, or with neutral co-lipids such as dioleoylphosphatidyl-ethanolamine, ("DOPE"), to facilitate delivery to cells of the biologically active molecules.

Now based on the disclosure contained herein, the present invention provides for the use of a plasmid comprising a gene of interest for the manufacture of a medicament for the treatment of a mammal by gene therapy, wherein the use comprises modifying the plasmid by removing or mutating to a non-CpG dinucleotide at least one CpG dinucleotide within said plasmid, and wherein the treatment comprises administering the plasmid in combination with a cationic amphiphile.

The present invention also provides for a process of preparing a pharmaceutical composition for use in gene therapy in a mammal, wherein the pharmaceutical composition comprises: (i) a plasmid comprising a gene of interest; and (ii) a cationic amphiphile; and wherein the process comprises: (a) modifying the plasmid by removing or mutating to a non-CpG dinucleotide at least one CpG dinucleotide within the plasmid; and (b) combining the plasmid with the cationic amphiphile.

### Brief Description of the Drawings

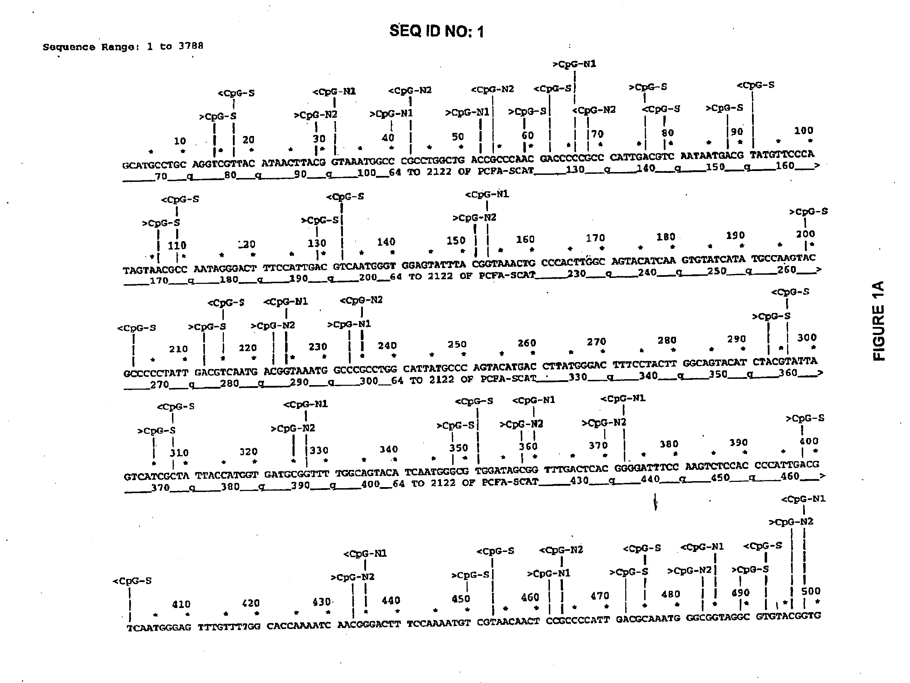

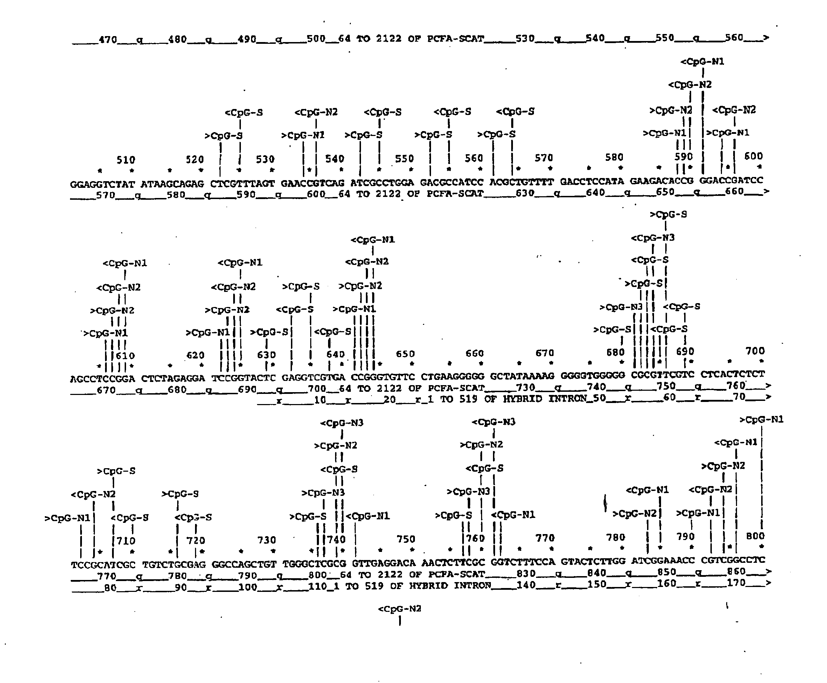

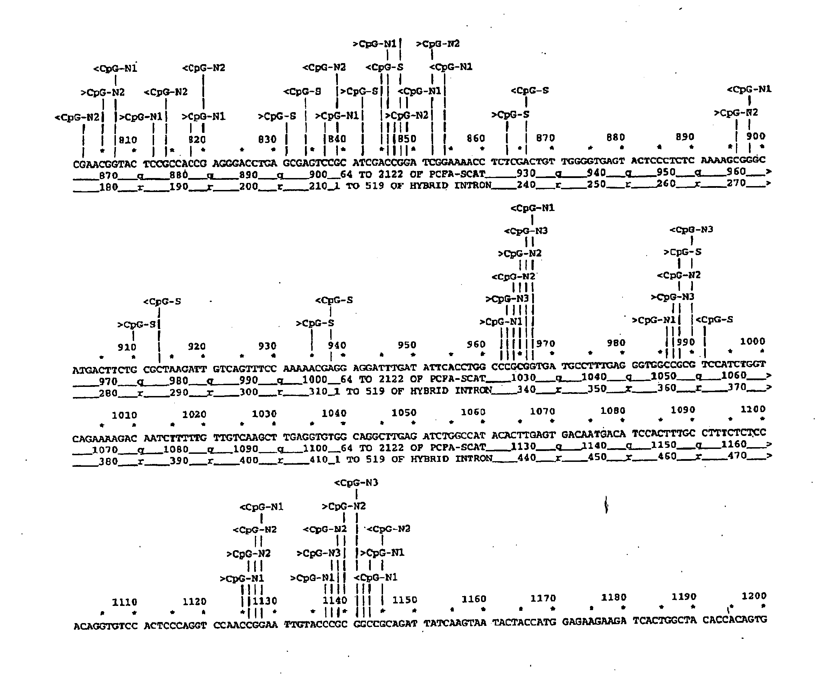

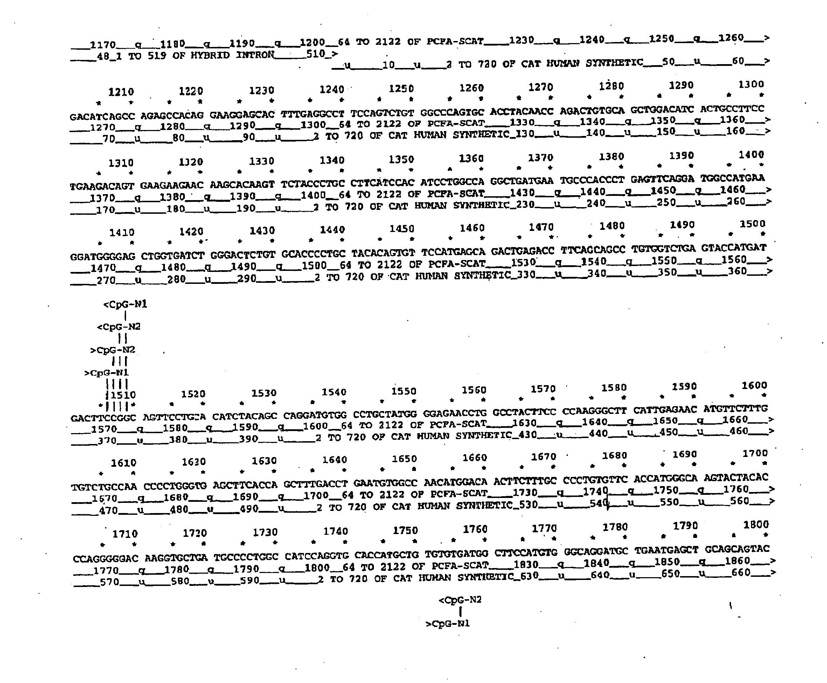

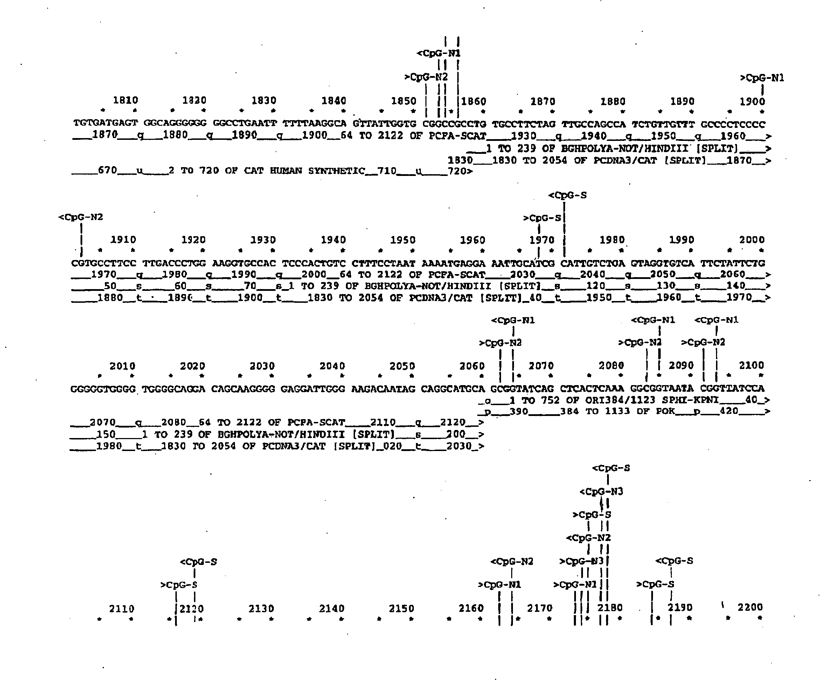

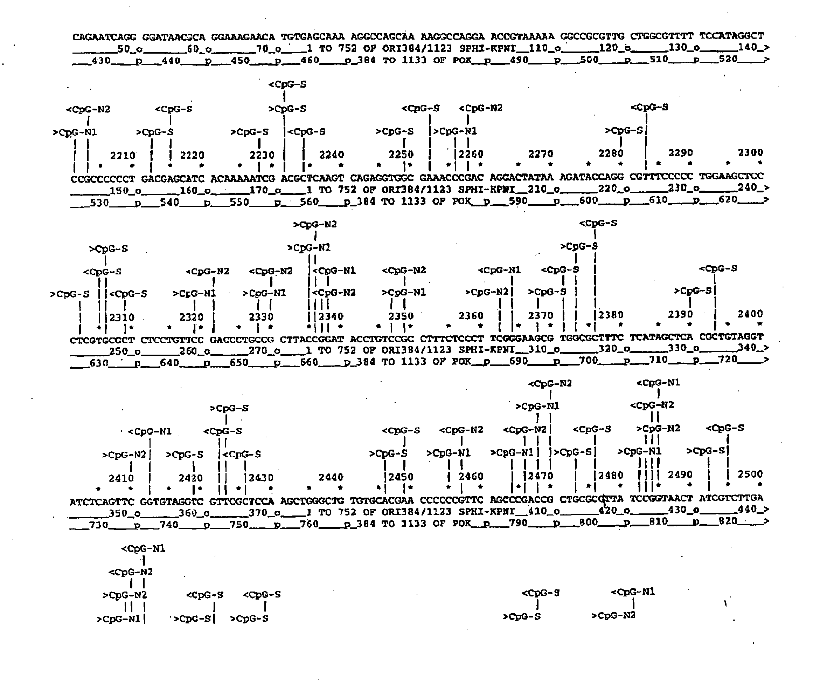

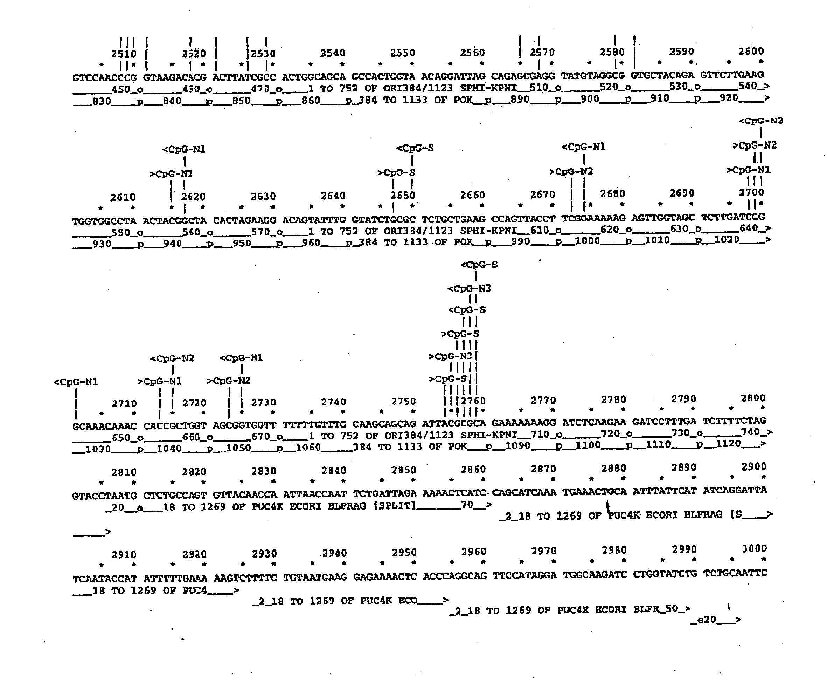

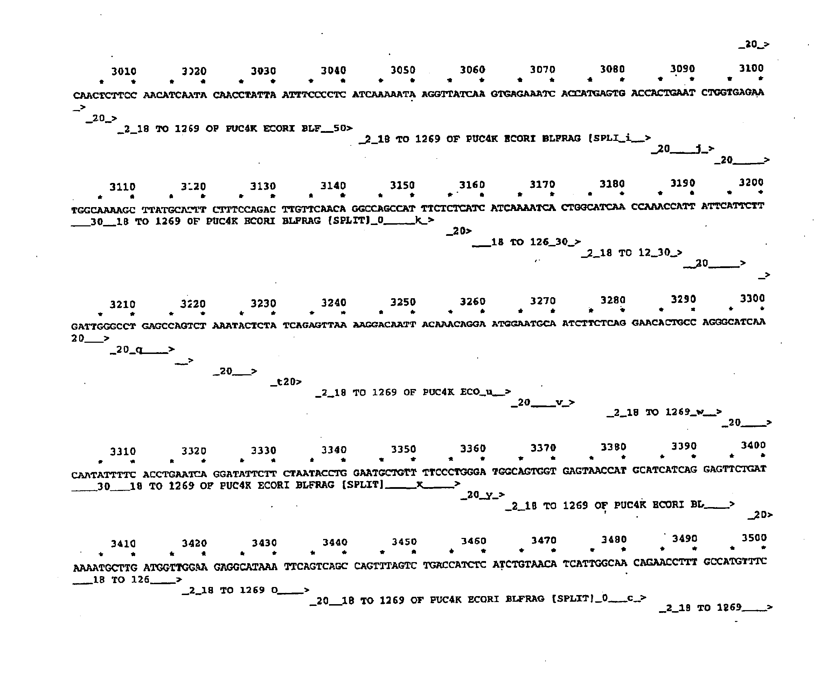

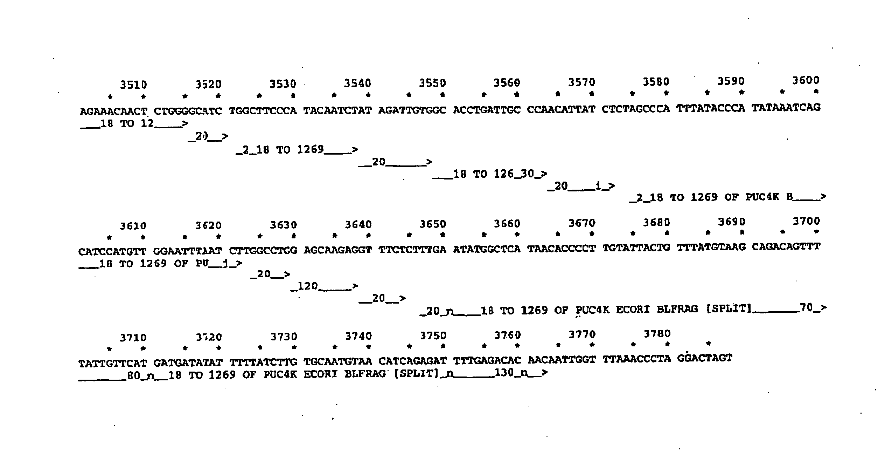

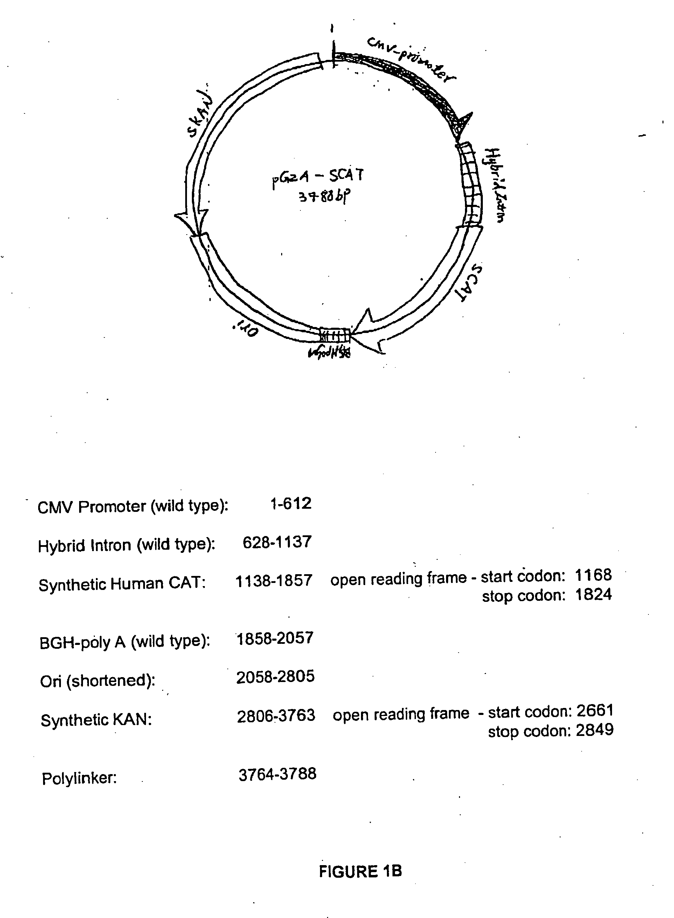

**Figure 1****.(a)** Nucleotide sequence of pGZA-CAT (also referred to as pGZA-sCAT) SEQ ID NO:1. (b) Map of nucleotide sequence pGZA-CAT.

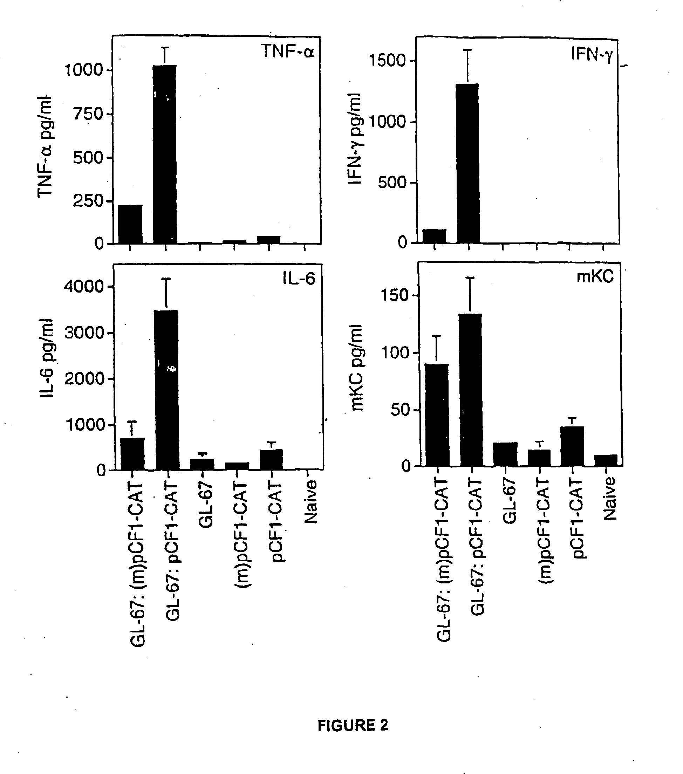

**Figure 2****.** Cytokine analysis of mouse BALF after instillation of GL-67 complexed with methylated or unmethylated pCF1-CAT. Groups of three BALB/c mice were instilled intranasally with 100 µl of GL-67:(m)pCF1-CAT, GL-67:pCF1-CAT, GL-67 alone, (m)pCF1-CAT, pCF1-CAT, or vehicle (naive). BALF was collected 24 h after instillation and ELISA assays were used to measure the levels of various cytokines. (m)pCF1-CAT refers to pCF1-CAT that had been methylated by Sss I methylase.

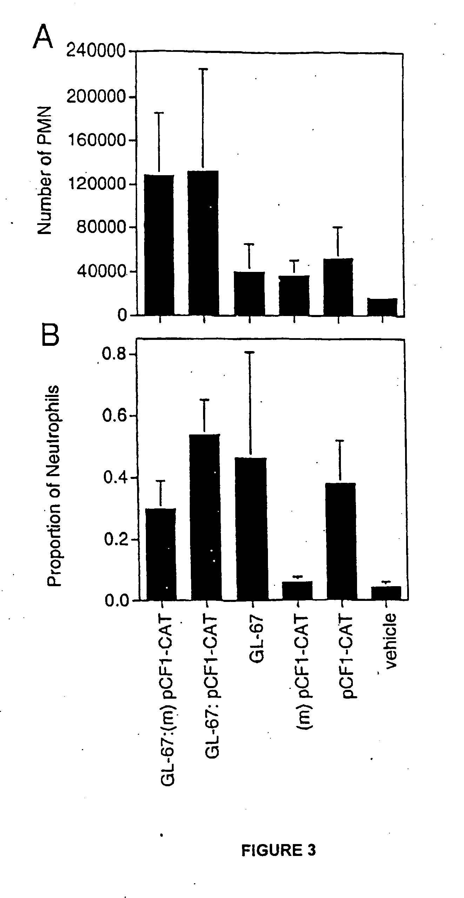

**Figure 3****.** Total cell counts and proportion of neutrophils in BALF after administration of cationic lipid:pDNA complexes. Groups of three BALB/c mice were instilled intranasally with 100 µl of GL-67:(m)pCF1-CAT, GL-67:pCF1-CAT, GL-67 alone, (m)pCF1-CAT, pCF1-CAT, or vehicle. BALF was collected 24 h post-instillation and total cells and the different cell types were counted. (m)pCF1-CAT refers to pCH-CAT that had been methylated by Sss I methylase. PMN, polymorphonuclear leukocytes.

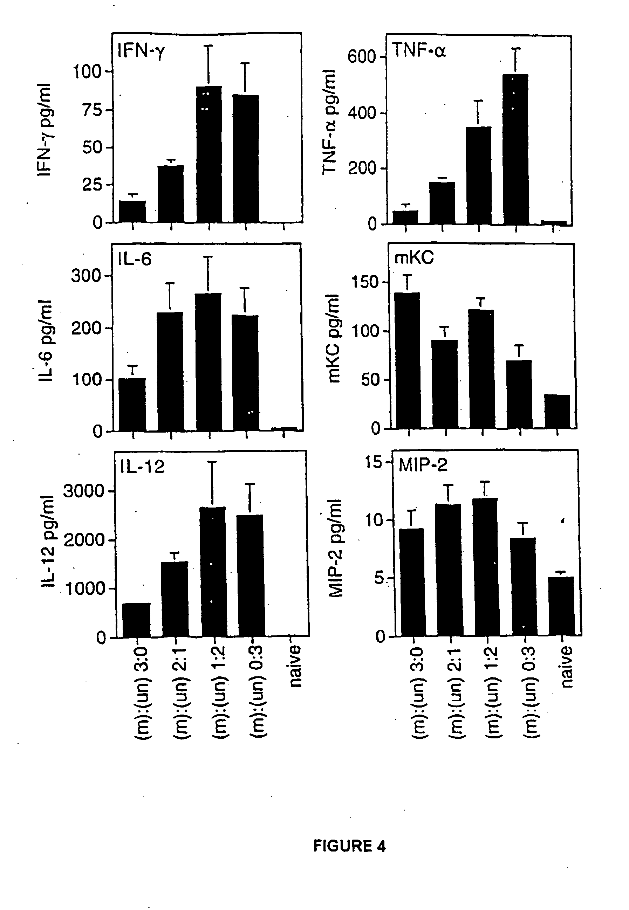

**Figure 4****.** Cytokine analysis of mouse BALF after instillation of GL-67 complexed with mixtures of methylated and unmethylated pCF1-CAT. Sss I-methylated pCF1-CAT was mixed with unmethylated pCF1-CAT at ratios of 0:3, 1:2, 2:1, or 3:0 [(m)pCF1-CAT:pCF1-CAT], then complexed with GL-67 to final concentration of 0.3:1.8 MM (GL-67:pDNA). Groups of three BALB/c mice were instilled intranasally with 100 µl of GL-67:pDNA complexes and BALF was collected 24 h after instillation for cytokine assays. Naive animals were treated with vehicle. (m), methylated pCF1-CAT; (un), non-methylated pCF1-CAT.

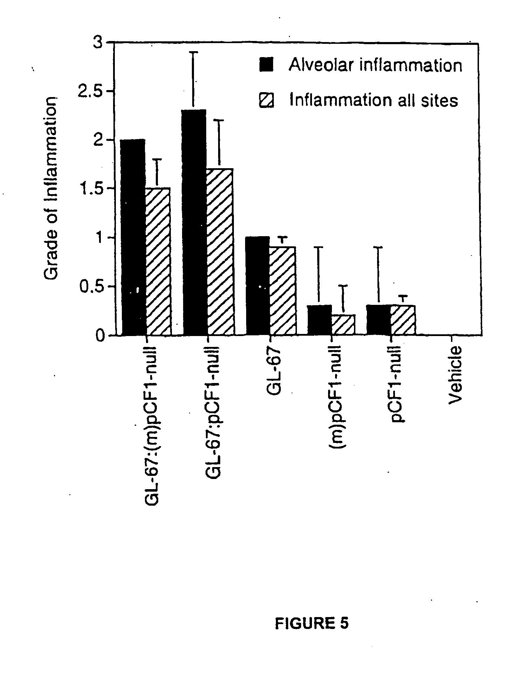

**Figure 5****.** Histopathological analysis of BALB/c mouse lung sections following administration of GL-67 complexed with methylated or unmethylated pCF1-CAT. BALB/c mice were instilled intranasally with 100 µl of GL-67:(m)pCF1-CAT, GL-67:pCF1-CAT, GL-67 alone, (m)pCF1-CAT, pCF1-CAT, or vehicle. Mice were sacrificed two days post-instillation and the lungs were processed for histological examination in a blinded manner. Lung inflammation was graded on a scale of 0 to 4, with 0 indicating no change, 1 a minimal change, 2 a mild change, 3 a moderate change, and 4 indicating a severe change from a normal lung. (m)pCF1-CAT refers to pCF1-CAT that had been methylated by Sss I methylase.

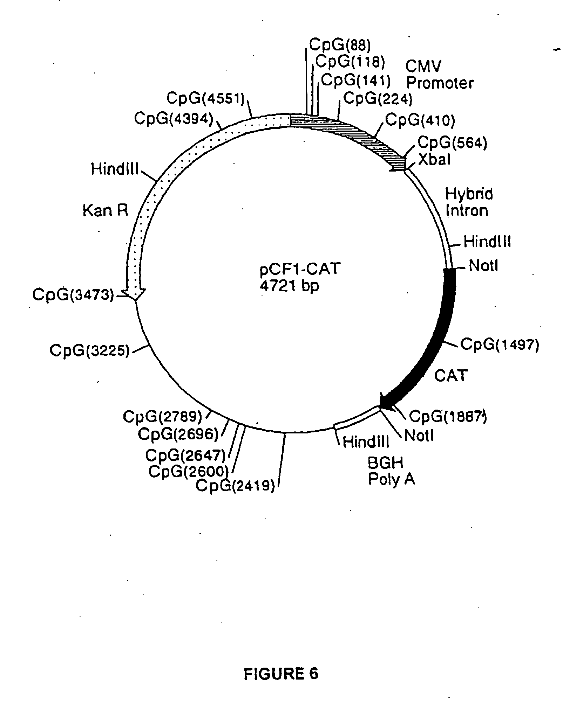

**Figure 6****.** CpG motifs present in pCF1-CAT. The motifs having the sequence 5'-RRCGYY-3' are as shown. Numbers in parentheses indicate the nucleotide position of the cytosine residue. Kan R, gene for kanamycin; CMV, cytomegalovirus; promoter, CAT, cDNA for chloramphenicol aceyltransferase; BGH PolyA, polyadenylation sequence from bovine growth hormone.

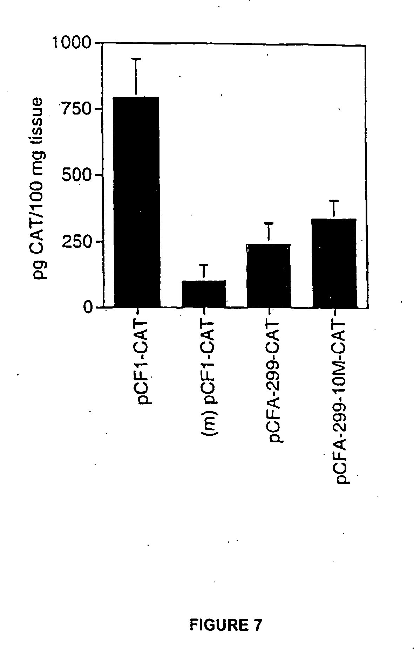

**Figure 7****.** Relative levels of CAT expression following methylation or mutagenesis of pCF1-CAT Groups of three BALB/c mice were instilled intranasally with 100 µl of GL-67:pCF1-CAT, GL-67:(m)pCF1-CAT, GL-67:pCFA-299-CAT, or GL-67:pCFA-299-10M-CAT. pCFA-299-CAT harbors a partial deletion of the CMV promoter and pCFA-299-10M-CAT, an additional 10 mutations at CpG sites harboring the sequence motif RRCGYY. (m)pCF1-CAT refers to pCF1-CAT that had been methylated by Sss I methylase. Lungs were harvested for CAT analysis at day 2 post-instillation.

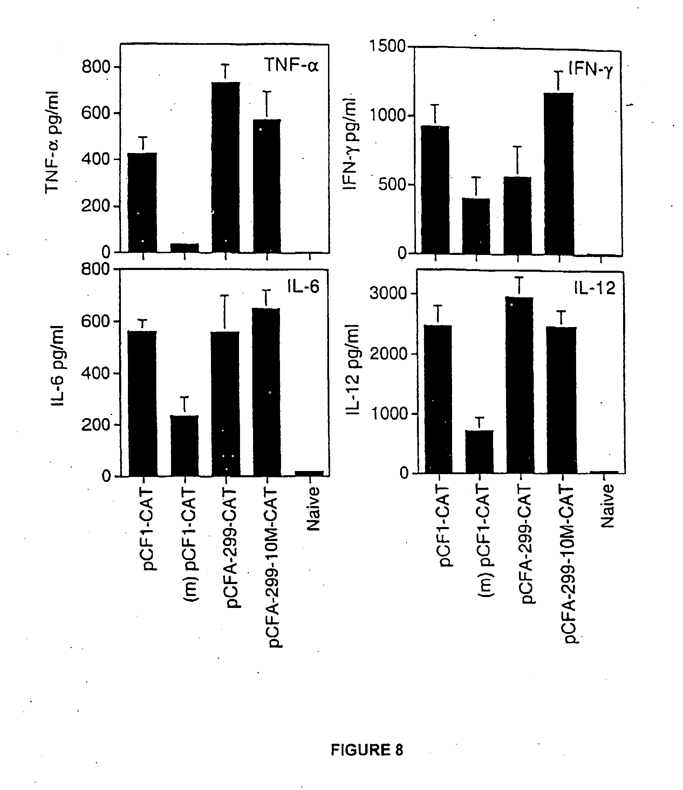

**Figure 8****.** Cytokine analysis of mouse BALF after instillation of GL-67 complexed with pCF1 -CAT and modified forms of pCF1-CAT containing reduced numbers of CpG motifs. Groups of three BALB/c mice were instilled intranasally with 100 µl of GL-67:pCF1-CAT, GL-67:(m)pCF1-CAT, GL-67:pCFA-299-CAT, or GL-67:pCFA-299-10M-CAT. BALF was collected 24 h after instillation and ELISA assays for TNF-α, IFN-γ, IL-6, and IL-12 were performed. (m)pCF1-CAT refers to pCF1-CAT that had been methylated by Sss I methylase. pCFA-299-CAT harbors a partial deletion of the CMV promoter and pCFA-299-10M-CAT, an additional 10 mutations at CpG sites harboring the sequence motif RRCGYY.

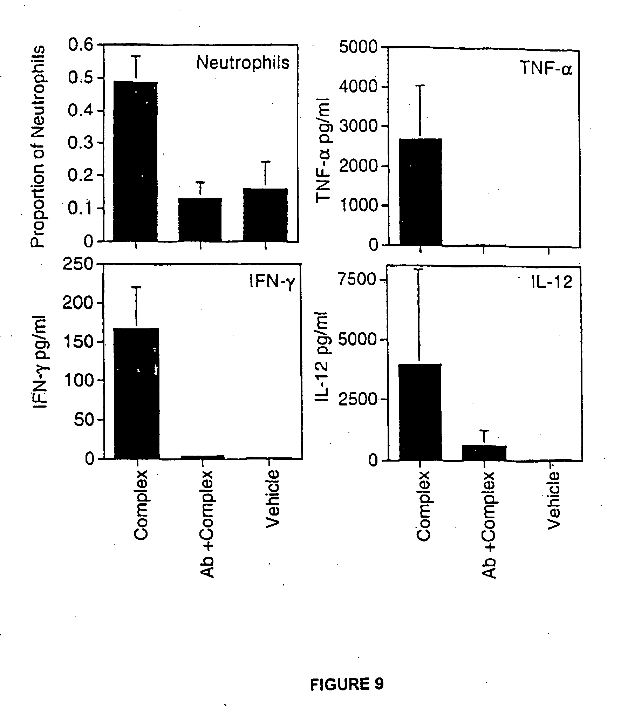

**Figure 9****.** Effect of inhibiting neutrophil influx on cytokine levels in the BALF of BALB/c mice. Animals were injected via the tail vein with a mixture of antibodies against murine LFA-1 and Mac-1α approximately 15 min prior to instillation of GL-67:pCF1-CAT into the lung. Mice were sacrificed 24 h post-instillation and BALF was collected for cell counts and cytokine quantization. Control mice received no antibody prior to instillation of complex, or were instilled with water (Vehicle). Ab refers to group that had been treated with the antibodies.

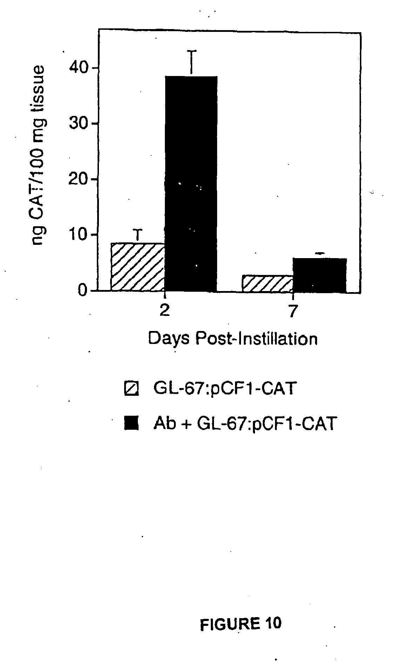

**Figure 10****.** Effect of inhibiting neutrophil influx on CAT expression in the lung. BALB/c mice were injected via the tail vein with a mixture of antibodies against murine LFA-1 and Mac-1α approximately 15 min prior to instillation of GL-67:pCF1-CAT into the lung. Mice were sacrificed 2 and 7 days post-instillation and the lungs assayed for CAT activity. Ab refers to group that had been treated with the antibodies.

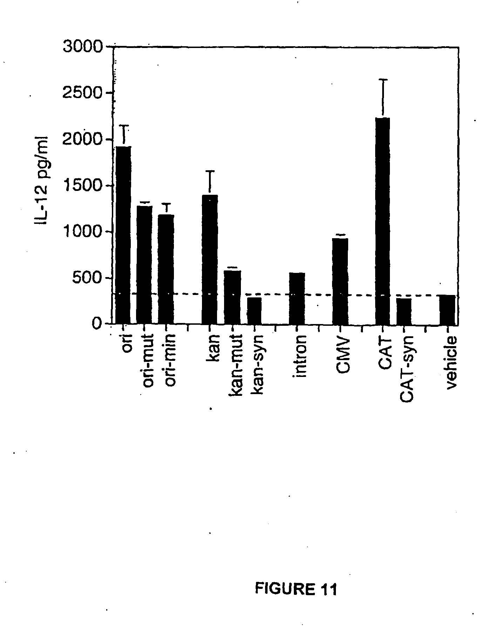

**Figure 11****.** IL-12 induction from mouse spleen cells by unmodified and mutated DNA fragments of pCFA-CAT. Fragments were amplified by PCR then each fragment was added to mouse spleen cells and the media was collected 24 hours later. IL-12 levels were assayed by ELISA. Vehicle is water. ori, replication origin region; ori-mut, mutated origin; ori-min, minimal origin; kan, kanamycin resistance gene. Data are expressed as mean ± SEM.

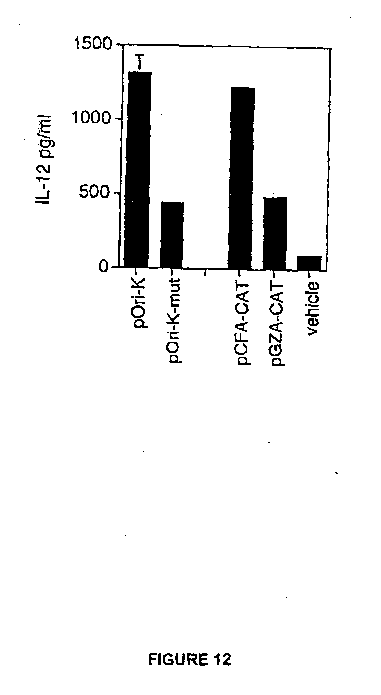

**Figure 12****.** IL-12 induction from mouse spleen cells by unmodified and mutated pDNA vectors. Plasmid DNA was added to mouse spleen cells and the media was collected 24 hours later. IL-12 levels were assayed by ELISA. Vehicle is water. Data are expressed as mean ± SEM.

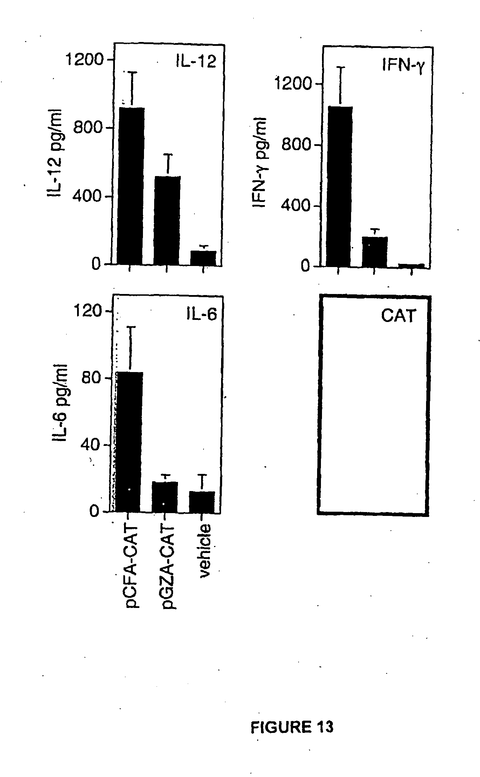

**Figure 13****.** Cytokine levels in serum and CAT expression in the lungs after intravenous administration of unmodified and mutated pDNA vectors. GL-62:pDNA complexes were injected via the tail vein into BALB/c mice. Serum was collected 24 hours post-instillation and IFN-γ, IL-12, and IL-6 levels were assayed by ELISA. Vehicle is water. Data are expressed as mean ± SEM.

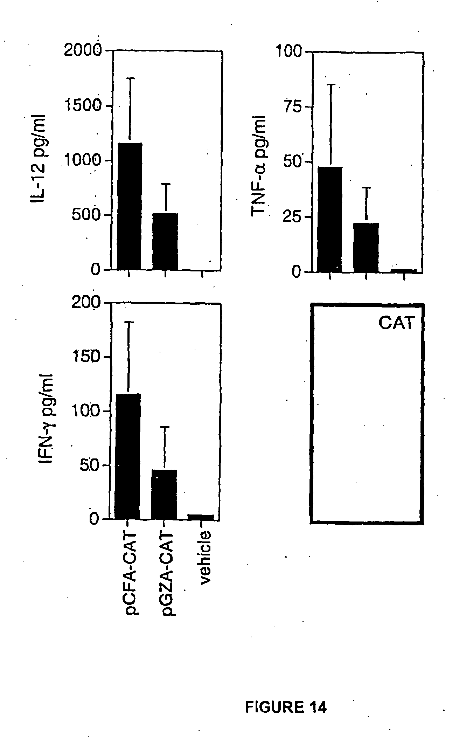

**Figure 14****.** Cytokine levels in bronchoalveolar lavage fluid (BALF) and CAT expression in the lungs after instillation of unmodified and mutated pDNA vectors. GL-67:pDNA complexes were instilled intranasally into the lungs of BALB/c mice. BALF was collected 24 hours post-instillation and IFN-γ, IL-12, and IL-6 levels were assayed by ELISA. Vehicle is water. Data are expressed as mean ± SEM.

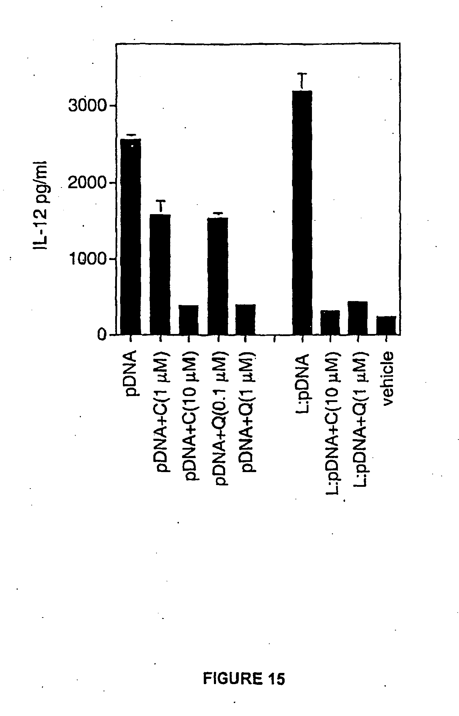

**Figure 15****.** Inhibition of IL-12 production from stimulated mouse spleen cells with chloroquine and quinacrine, pCFA-CAT (pDNA) or GL-67:pCFA-CAT (L:pDNA) plus or minus chloroquine (C) or quinacrine (Q) were added to mouse spleen cells and the media was collected 24 hours later. Vehicle is water. Data are expressed as mean ± SEM.

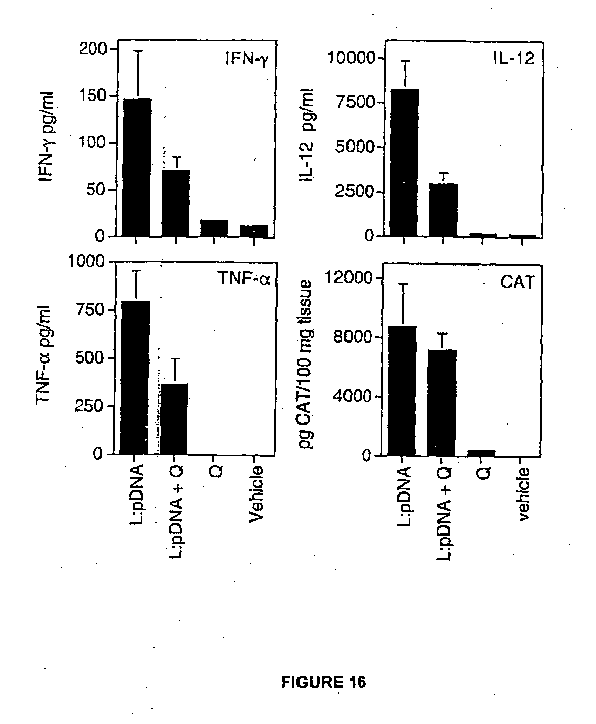

**Figure 16****.** Cytokine levels in bronchoalveolar lavage fluid after instillation of lipid:pDNA complex plus chloroquine or quinacrine. IL-12 levels were assayed by ELISA. Data are expressed as mean ±SEM.

### Detailed Description of the Invention

According to the present disclosure, a plasmid may be modified to reduce the inflammatory response to the plasmid to both reduce toxicity and increase the efficacy of gene delivery. The plasmid may also be modified in order to modulate a mammal's immunostimulatory response to a plasmid composition. The modified plasmid may be administered alone, as the active ingredient in a formulation, or as part of a complex with a carrier such as lipids, including cationic amphiphile compounds, viral vectors, including adenoviruses, and other methods that have been employed in the art to effectuate delivery of biologically active molecules into the cells of mammals. The plasmid:carrier complexes may also be administered alone or as the active ingredient in a formulation. These elements will now be discussed.

The present disclosure provides for the use of a modified plasmid with any of the methods that effectuate delivery of biologically active molecules into the cells of mammals. In a preferred aspect, however, modified plasmid DNA is used with a cationic amphiphile or a viral formulation such as a viral vector or an adenovirus.

In a preferred aspect, the present disclosure provides for the use of any cationic amphiphile compounds, and compositions containing them, that are useful to facilitate delivery of modified plasmid DNA. Amphiphiles that are particularly useful facilitate the transport of biologically active polynucleotides into cells, and in particular to the cells of patients for the purpose of gene therapy.

Now as discussed alsewhere herein, the present invention provides for the use of a plasmid comprising a gene of interest for the manufacture pf a medicament for the treatment of a mammal by gene therapy, wherein the use comprises modifying the plamid by removing or mutating to a non-CpG dinucleotide at least one CpG dinucleotide within said plasmid, and wherein the treatment comprises administering the plasmid in combination with a cationic amphiphile.

In addition, the present invention provides for a process of preparing a pharmaceutical composition for use in gene therapy in a mammal, wherein the pharmaceutical composition comprises: (i) a plasmid comprising a gene of interest; and (ii) a cationic amphiphile; and wherein the process comprises: (a) modifying the plasmid by removing or mutating to a non-CpG dinucleotide at least one CpG dinucleotide within the plasmid; and (b) combining the plasmid with the cationic amphiphile.

A number of preferred cationic amphiphiles according to the practice of the present disclosure, including the present invention, can be found in U.S. Patents No. 5,747,471 & 5,650,096 and PCT publication WO 98/02191. In addition to cationic amphiphile compounds, these two patents disclose numerous preferred co-lipids, biologically active molecules, formulations, procedures, routes of administration, and dosages. The disclosures of which have been specifically incorporated by reference herein.

In connection with the practice of the present disclosure, including the present invention, cationic amphiphiles tend to have one or more positive charges in a solution that is at or near physiological
pH. Representative cationic amphiphiles that are useful in the practice of the present disclosure, including the present invention, are: 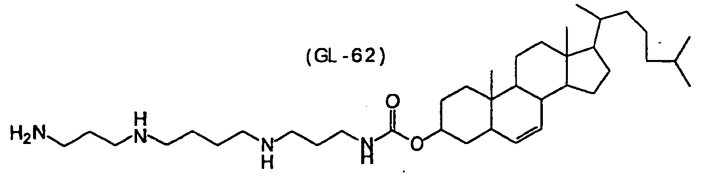 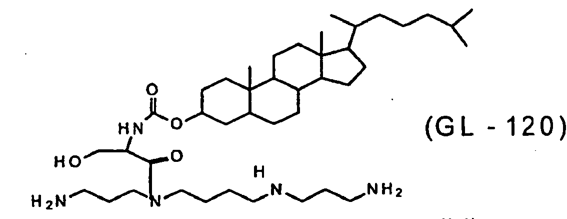 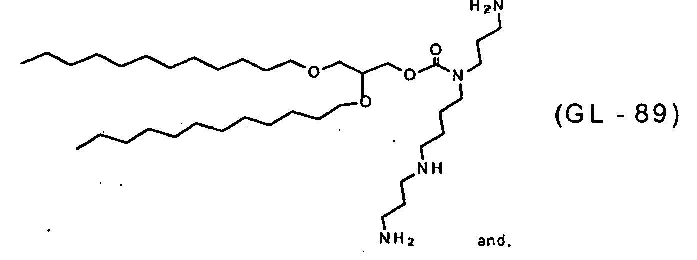 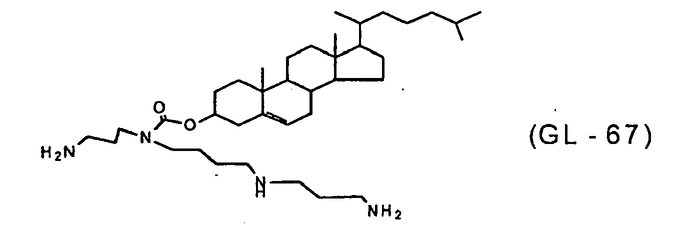 and other amphiphiles that are known in the art including those described in U.S. Patents No. 5,747,471 & 5,650,096 and PCT publication WO 98/02191.

It has been determined that the stability and transfection-enhancing capability of cationic amphiphile compositions can be substantially improved by adding to such formulations small additional amounts of one or more derivatized polyethylene glycol compounds. Such enhanced performance is particularly apparent when measured by stability of cationic amphiphile formulations to storage and manipulation, including in liquid (suspended) form, and when measured by stability during aerosol delivery of such formulations containing a therapeutic molecule, particularly polynucleotides.

According to the practice of the present disclosure, including the present invention, any derivative of polyethylene glycol may be part of a cationic amphiphile formulation. Complexes have been prepared using a variety of PEG derivatives and all of the PEG derivatives, at a certain minimum cationic amphiphile:PEG derivative ratio have been able to form stable homogeneous complexes.

PEG derivatives stabilize cationic amphiphile formulations and enhance the transfecting properties and the affinity of formulations to biologically active molecules. The use of PEG and PEG derivatives enables one to use a higher ratio of DNA to lipids. Previous attempts to prepare more concentrated lipid:pDNA complexes using resulted in precipitation of the complexes, especially at lipid:pDNA ratios for which the majority of the pDNA was bound to lipid. It was believed that the precipitation observed at higher concentrations might be related to a phase separation of the cationic lipid component from the non-bilayer lipid component. In an effort to maintain the traditional lipid formulations in a bilayer configuration, PEG-containing lipids were found to be effective in preventing precipitation of the complex at higher pDNA concentrations.

Only a small mole fraction of PEG-containing lipid was found to be required to form stable formulations that did not precipitate at high concentrations of lipid and DNA. For example, at 0.05 mol PEG-DMPA, cationic lipid:pDNA complexes could be stabilized at pDNA concentrations exceeding 20 mM. Without PEG-containing lipids, a salt excipient, which is not preferred since an ionic excipient also depresses transfection activity, is required to maintain the lipid: pDNA ratio of the complex during certain methods such as aerosolization. For more information regarding use of PEG derivatives the following references are specifically incorporated by reference. Simon J. Eastman et al., Human Gene Therapy, 8, pp. 765-773 (1997); Simon J. Eastman et al. Human Gene Therapy, p. 8, pp. 313-322 (1997).

Derivatives of polyethylene glycol useful in the practice of the present disclosure, including the present invention include any PEG polymer derivative with a hydrophobic group attached to the PEG polymer. Examples would include PEG-PE, PEG-DMPE, PEG-DOPE, PEG-DPPE, or PEG-Serinamide. Not to be limited as to theory, it is believed that preferred PEG-containing lipids would be any PEG polymer derivatives attached to a hydrophobic group that can anchor to the cell membrane. Two highly preferred species thereof include dimyristoylphosphatidylethanolamine (di C₁₄) ("DMPE") and dilaurylphosphatidylethanolamine (di C₁₂) ("DLPE").

With respect to selection of the PEG polymer, it is a preferred aspect of the present disclosure, and a preferred embodiment of the invention that the polymer be linear, having a molecular weight ranging from 1,000 to 10,000. Preferred species thereof include those having molecular weights from 1500 to 7000, with 2000 and 5000 being examples of useful, and commercially available sizes. In the practice of the present disclosure, including the present invention, it is convenient to use derivatized PEG species provided from commercial sources, and it is noted that the molecular weight assigned to PEG in such products often represents a molecular weight average, there being shorter and longer molecules in the product. Such molecular weight ranges are typically a consequence of the synthetic procedures used, and the use of any such product is within the practice of the present disclosure, including the present invention.

It is also within the practice of the present disclosure, including the present invention, to use derivatized-PEG species that (1) include more than one attached phospholipid, or (2) include branched PEG sequence, or (3) include both of modifications (1) and (2).

Accordingly, preferred species of derivatized PEG include:
(a) polyethylene glycol 5000-dimyristoylphosphatidylethanolamine, also referred to as PEG₍₅₀₀₀₎-DMPE;
(b) polyethylene glycol 2000-dimyristoylphosphatidylethanolamine, also referred to as PEG₍₂₀₀₀₎-DMPE);
(c) polyethylene glycol 5000-dilaurylphosphatidylethanolamine, also referred to as PEG₍₅₀₀₀₎-DLPE); and
(d) polyethylene glycol 2000-dilaurylphosphatidylethanolamine, also referred to as PEG₍₂₀₀₀₎-DLPE).

Certain phospholipid derivatives of PEG may be obtained from commercial suppliers. For example, the following species: di C14:0, di C16:0, di C18:0, di C18:1, and 16:0/18:1 are available as average 2000 or average 5000 MW PEG derivatives from Avanti Polar Lipids, Alabaster, AL, USA, as catalog nos. 880150, 880160, 880120, 880130, 880140, 880210, 880200, 880220, 880230, and 880240.

The use of neutral co-lipids is optional. Depending on the formulation, including neutral co-lipids may substantially enhance delivery and transfection capabilities. Representative neutral co-lipids include dioleoylphosphatidylethanolamine ("DOPE"), the species most commonly used in the art, diphytanoylphosphatidylethanolamine, lysophosphatidylethanolamines other phosphatidyl-ethanolamines, phosphatidylcholines, lyso-phosphatidylcholines and cholesterol. Typically, a preferred molar ratio of cationic amphiphile to co-lipid is about 1:1. However, it is within the practice of the present disclosure, including the present invention, to vary this ratio, including also over a considerable range, although a ratio from 2:1 through 1:2 is usually preferable. Use of diphytanoylphosphatidylethanolamine is highly preferred according to the practice of the present disclosure, including the present invention, as is use of "DOPE".

According to the practice of the present disclosure, including the present invention, preferred formulations may also be defined in relation to the mole ratio of PEG derivative, however, the preferred ratio will vary with the cationic amphiphile chosen. In preferred examples thereof, the neutral co-lipid is diphytanoylphosphatidylethanolamine, or is DOPE, and the PEG derivative is a DMPE or DLPE conjugate of PEG₂₀₀₀ or PEG₅₀₀₀. In a highly preferred example, the neutral co-lipid is diphytanoylphosphatidylethanolamine, and the PEG derivative is PEG₍₂₀₀₀₎-DMPE.

The present disclosure provides a method to modulate a mammal's immunostimulatory response to a composition and both reduce the toxicity and increase the efficacy of gene delivery. In the practice of the present disclosure, plasmid DNA may be modified to reduce the inflammatory response to the plasmid DNA. In one aspect, CpG motifs of plasmid DNA may be methylated to reduce the immunostimulatory response. In another aspect, CpG motifs of plasmid DNA may be removed to reduce the immunostimulatory response.

Plasmid DNA contributes significantly to the inflammatory response observed following administration of cationic lipid:pDNA complexes to the lung. Most of the increase in the levels of the cytokines TNF-α, IFN-γ, IL-12 and IL-6 and a proportion of the cellular influx observed in the BALF following delivery of cationic lipid:pDNA complexes was shown to be attributable to the pDNA. Not to be limited as to theory, the basis for this inflammatory response was determined to be due to the presence of unmethylated CpG dinucleotides in the pDNA. The involvement of the CpG dinucleotides was implicated from experiments demonstrating that the inflammatory response could be abated by methylating the pDNA with a CpG methylase. Furthermore, a dose-response relationship was attained between the amount of unmethylated pDNA used in the instillation and the levels of cytokines induced.

Induction of the inflammatory response by unmethylated pDNA was significantly exacerbated upon complexation with a cationic lipid. Not to be limited as to theory, this enhanced response was likely due to either increased cellular uptake of the pDNA by the cells or increased stabilization of the pDNA by the cationic lipid in the intracellular compartment.
Consistent with this proposal is the observation that CpG oligonucleotides covalently linked to a solid support such that they are no longer internalized, are nonstimulatory.

The induction of cytokines and the increase in a proportion of the cellular infiltrates, particularly of neutrophils in the BALF, is believed to be the effect of the pDNA component. That a reduction in neutrophil concentration in the BALF by administration of antibodies to Mac-1α and LFA-1 was coincident with a concomitant decline in cytokine levels is consistent with this proposal. Accordingly, some of the observed inflammatory response resulted as a direct manifestation of the complexed cationic lipid:pDNA. Examples of these include activation of the cytokine KC and recruitment of other cells into the BALF such as macrophages and lymphocytes to presumably clear the relatively large particulates of complexed cationic lipid:pDNA.

Strategies to minimize the immunostimulatory effects of CpG dinucleotides in pDNA are within the practice of the present disclosure. In one aspect of the present disclosure, methylation of the CpG motifs suppresses the inflammation in the lung. Indeed, it has been known that methylation of CpG is associated with long-term inactivation of certain genes during mammalian development and in the repression of viral genomes. In this regard, selection of promoters that lack CpG motifs and are therefore insensitive to methylation represent an additional aspect of the present disclosure. One such known promoter is the MMTV-LTR (murine moloney tumor virus-long terminal repeat).

It is also within the practice of the present disclosure to modulate the immunostimulatory properties of pDNA by altering the CpG content of a plasmid. The CpG content of a plasmid may be altered by increasing or decreasing the presence of CpG motifs, or by modifying the CpG motifs which are present by increasing or decreasing the amount of methylation. Increased immunostimulation may be achieved by increasing the number of CpG motifs present in the plasmid or by reducing the amount of methylation for such CpG motifs. Increasing the number of CpG motifs combined with reduced methylation sites may prove particularly useful if immunostimulation is desired.

*In vitro* methylation of all the CpG dinuoleotides within a given pDNA has been shown to significantly decrease cytokine induction, but may also severely inhibit transgene expression. Elimination of CpG motifs can be achieved by deleting non-essential regions of the vector. DNA fragments containing only the promoter, transgene, and polyadenylation signal have been shown to have decreased stimulatory activity after intravenous delivery into mice. Within the practice of the present disclosure is a method of eliminating some non-essential regions, while retaining a functional origin and antibiotic resistance gene. Site-directed mutagenesis and synthetic fragments devoid of CpG sequences may be used to generate a less stimulatory vector.

In accordance with the present disclosure, a pDNA expression vector may be constructed containing substantially fewer CpG dinculeotides within its sequence than conventional plasmids. The reduced CpG content has been shown to correlate with a decreased immunostimulatory response *in vitro,* and cationic lipid-pDNA complexes containing these modified plasmids induced significantly lower levels of proinflammatory cytokines in the serum when administered intravenously, as well as decreased levels in the mouse lung when administered intranasally. The role of CpG content was demonstrated by the observation that DNA fragments lacking CpG dinucleotides were non-stimulatory both *in vitro* and *in vivo.*

Prior toxicology studies have indicated that the physiological changes mediated by systemic administration of cationic lipid:pDNA complexes are also characterized in part by statistically significant elevations in serum transaminase (ALT, AST) levels. Elevations in serum transaminase (ALT, AST) levels are generally accepted as indicators of liver toxicity, although other clinical measures of liver damage are certainly recognized and could be substituted. It is therefore within the practice of the present disclosure to measure the immunostimulatory response of a mammal by monitoring liver enzyme levels such as AST and ALT levels. In one aspect, a desired immunostimulatory response is obtained by altering the CpG content of a plasmid and monitoring the liver enzyme levels in the blood until the desired immunostimulatory response is observed.

It is also within the practice of the present disclosure to measure the immunostimulatory response of a mammal by monitoring the cytokine levels in the mammal. In one aspect, a desired immunostimulatory response is obtained by altering the CpG content of a plasmid and monitoring the cytokine levels in the blood until the desired immunostimulatory response is observed.

Another strategy to modulate the immunostimulatory properties of the pDNA vector is to use specific inhibitors of the CpG signaling pathway. Uptake of DNA into an acidified intracellular compartment via endocytosis is the first required step in the pathway. Inhibitors of endosomal acidification such as monensin, bafilomycin, chloroquine, and quinacrine may effectively block CpG induced cytokine induction by leukocytes in vitro.

A distinctive property of chloroquine or quinacrine are the low concentrations required for the specific inhibition of CpG mediated stimulation. An effective dose in the mouse lung is a comparatively small dose in the human lung. A dose of 0.5 micrograms chlorocrine in the mouse lung is equivalent to approximately 1.0 mg in the human lung. Given the limitations of such extrapolations, the dose nevertheless compares favorably to the recommended dosage for antimalarial indications (approximately 100-200mg).

The present disclosure also provides for a method of genetically altering those CpG motifs that have been shown to exhibit potent immunostimulatory activity, such as the most immunostimulatory motif 5'- RRCGYY-3'. However, other CpG dinucleotides that are not within the sequence context of RRCCGY also contribute to the cytokine induction. Removal by site-directed mutagenesis of these sites is preferred.

In yet another aspect, CpG motifs that exhibit neutralizing activity are used to counter those with immunostimulatory activity. As such, the incorporation of these neutralizing motifs coupled with the removal of those exhibiting immunostimulatory activity from pDNA vectors will reduce the inflammatory response in the lung.

Compositions containing CpG altered plasmids are also within the practice of the present disclosure. In one aspect, a composition comprises the CpG altered plasmid, pGZA-CAT as shown in Figure 1, SEQ ID NO:1. The plasmid may be CpG altered by site-directed mutagenesis.

An alternate approach to mutagenesis for reducing the number of CpG sites within the replication origin region was to determine the minimal fragment that could still be functional. Another aspect of the present disclosure is a composition comprising a selectable marker or fragment of the CpG altered plasmid, such as a selectable marker of pGZA-CAT. Representative fragments include those depicted in Table 1 and in Figure 1.

The decrease in the stimulatory activity of the pDNA vector was found to be roughly proportional to the number of CpG sites. This mimics the strategy of CpG suppression found in vertebrate DNA, but vertebrate DNA also contains other features within its sequence that reduce its stimulatory properties. CpG motifs in particular sequence contexts have been shown to be non-stimulatory and have been termed neutralizing (CpG-N) motifs. Oligonucleotides containing certain patterns of CGG, CCG, and CGCG direct repeat motifs not only lack stimulatory activity but they can also inhibit stimulatory CpG motifs *in cis* and *in trans.* Although potentially useful, inserting additional CpG-N motifs into the pDNA vector has so far not altered stimulatory activity. The interactions between CpG-N and CpG -S motifs are not well understood, and at present the most effective strategy has been simply to reduce the number of CpG sites.

The remaining problematic CpG sites reside within the enhancer-promoter and replication origin region. An enhancer-promoter containing fewer CpG sites than found in CMV could be used in its place. The replication origin region, along with the antibiotic resistance gene could be deleted entirely. Site-specific recombination using a phage lambda integrase has been demonstrated to produce "minicircles" composed only of the expression cassette and a fragment of the recombined site. The purification of these recombined plasmids is at present only suitable for small-scale analytical purposes, but large-scale methods are certainly conceivable.

**Table 1. CpG sites in unmodified and mutated DNA fragments**

| | length (bp) | No. of CpG |
|---|---|---|
| origin wt | 1276 | 161 |
| origin mut | 1276 | 153 |
| origin min | 740 | 96 |
| | | |
| kan wt | 1252 | 117 |
| kan mut | 1252 | 85 |
| kan syn | 957 | 0 |
| | | |
| promoter wt | 607 | 74 |
| | | |
| intron/polyA wt | 734 | 80 |
| | | |
| CAT wt | 793 | 80 |
| CAT syn | 703 | 2 |
| | | |
| pCFA-CAT | 4739 | 526 |
| pGZA-CAT | 3788 | 256 |

To summarize, a pDNA vector containing substantially reduced numbers of CpG sites decreases the inflammatory response to the vector as well as to cationic lipid-pDNA complexes. One cautionary note is the report that exogenous DNA, regardless of CpG content, can upregulate MHC I expression in non-immune cells. Nevertheless, a pDNA with decreased immunostimulatory properties is a useful step toward increasing the safety and viability of non-viral gene therapy.

### Preparation of Pharmaceutical Compositions and Administration Thereof

The present disclosure provides for pharmaceutical compositions that facilitate intracellular delivery of therapeutically effective amounts of pDNA molecules. Pharmaceutical compositions of the present disclosure facilitate entry of pDNA into tissues and organs such as but not limited to the gastric mucosa, heart, lung, muscle and solid tumors.

In addition to pDNA, other representative biologically active molecules that can be provided intracellularly in therapeutic amounts using the methods of the present disclosure include: (a) polynucleotides such as genomic DNA, cDNA, and mRNA that encode for therapeutically useful proteins as are known in the art; (b) ribosomal RNA; (c) antisense polynucleotides, whether RNA or DNA, that are useful to inactivate transcription products of genes and which are useful, for example, as therapies to regulate the growth of malignant cells; (d) ribozymes; and
(e) low molecular weight biologically active molecules such as hormones and antibiotics.

Cationic amphiphile species, PEG derivatives, and co-lipids of the present disclosure may be blended so that two or more species of cationic amphiphile or PEG derivative or co-lipid are used, in combination, to facilitate entry of biologically active molecules into target cells and/or into subcellular compartments thereof. Cationic amphiphiles of the present disclosure can also be blended for such use with amphiphiles that are known in the art. Additionally, a targeting agent may be coupled to any combination of cationic amphiphile, PEG derivative, and co-lipid or other lipid or non-lipid formulation that effectuates delivery of a biologically active molecule to a mammalian cell.

Dosages of the pharmaceutical compositions of the present disclosure will vary, depending on factors such as half-life of the biologically-active molecule, potency of the biologically-active molecule, half-life of the delivery vehicle, any potential adverse effects of the delivery vehicle or of degradation products thereof, the route of administration, the condition of the patient, and the like. Such factors are capable of determination by those skilled in the art.

A variety of methods of administration may be used to provide highly accurate dosages of the pharmaceutical compositions of the present disclosure. Such preparations can be administered orally, parenterally, topically, transmucosally, or by injection of a preparation into a body cavity of the patient, or by using a sustained-release formulation containing a biodegradable material, or by onsite delivery using additional micelles, gels and liposomes. Nebulizing devices, powder inhalers, and aerosolized solutions are representative of methods that may be used to administer such preparations to the respiratory tract.

Additionally, the therapeutic compositions of the present disclosure can in general be formulated with excipients (such as the carbohydrates lactose, threose, sucrose, mannitol, maltose or galactose, and inorganic or organic salts) and may also be lyophilized (and then rehydrated) in the presence of such excipients prior to use. Conditions of optimized formulation for each complex of the present disclosure are capable of determination by those skilled in the pharmaceutical art. Selection of optimum concentrations of particular excipients for particular formulations is subject to experimentation, but can be determined by those skilled in the art for each such formulation.

### Examples

The following Examples are representative of the practice of the present disclosure.

### Example 1 Construction and purification of plasmid DNA.

The construction and characterization of the plasmid vector pCF1-CAT encoding the reporter gene product chloramphenicol acetyltransferase (CAT) has been described previously. See Yew et al. Hum. Gene Ther., 8, 575-84 (1997). pCF1-CAT contains the strong promoter from the human cytomegalovirus immediate-early gene (CMV), an intron, the bovine growth hormone polyadenylation signal sequence, a pUC origin, and the aminoglycoside 3'-phosphotransferase gene that confers resistance to kanamycin. pCF1-null is analogous to pCF1-CAT except that the cDNA for CAT was deleted. pCFA-299-CAT was constructed by digesting pCFA-CAT (identical to pCF1-CAT except for the addition of a small polylinker 5' of CMV) with Pme I (in the polylinker) and BgII (in CMV), blunting the ends with the Klenow fragment of DNA polymerase 1, then replicating. This results in deletion of nucleotides -522 to -300 of the CMV promoter.

Site-directed mutagenesis was performed using the QuickChange Site-Directed Mutagenesis kit (Stratagene) following the protocol described by the manufacturer. One modification was that multiple sets of oligonucleotides were used simultaneously, allowing mutagenesis of three or more sites in a single reaction. The mutations were confirmed by extensive DNA sequencing and restriction enzyme mapping to check for plasmid integrity. pCFA-299-10M-CAT is deleted of the CpG motifs at nucleotides 88, 118, 141, and 224 (number refers to the C residue within the CpG dinucleotide except where indicated and is based on the pCF1-CAT sequence; see Figure 6), and contains 10 point mutations at nucleotides 410, 564, 1497 (G to A), 1887, 2419, 2600, 2696, 3473, 4394 (G to A), and 4551.

Plasmid DNA was prepared by bacterial fermentation and purified by ultrafiltration and sequential column chromatography essentially as described previously. See Lee ef al., Hum. Gene Ther., 7, 1701-1717 (1996); Scheule et al., Hum. Gene Ther., 8, 689-707 1997). The purified preparations contained less than 5 endotoxin units/mg of pDNA as determined by a chromogenic LAL assay (BioWhiftaker, Maryland), less than 10 µg protein/mg pDNA as determined by the micro BCA assay (Pierce, Illinois), and less than 10 µg of bacterial chromosomal DNA/mg of pDNA as determined by a dot-blot assay. They were also essentially free of detectable RNA and exhibited spectrophotometric A_{260/280} ratios of between 1.8 and 2.0.

### Example 2 In vitro methylation of pDNA.

Plasmid DNAs were methylated *in vitro* in a 5 ml reaction containing 1 x NEB buffer 2 [50 mM NaCl, 10 mM Tds-HCl, pH 7.9, 10 MM MgCl₂, 1 mM dithiothreitol], 160 µM S-adenosylmethionine (SAM), 1-3 mg of pDNA, and 1 U of Sss I methylase (New England Biolabs) per µg of pDNA. The mixture was incubated at 37°C for 18 h. Additional SAM was added to a concentration of ISO µM after 4 h of incubation. Mock treatment of pDNA used the same procedure except the Sss I methylase was omitted. Methylated and mock-treated pDNA was centrifuged through a Millipore Probind column, ethanol precipitated, and washed with 70% (v/v) ethanol. The pDNA was resuspended in water to a final concentration of approximately 3 mg/ml. In experiments to examine the effects of Sss I-mediated methylation of pDNA, mock-methylated pDNA was always used as a control.

The extent of pDNA methylation was assessed by digesting 0.2-0.5 µg of the treated pDNA with 10 U BstU I or Hpa II for 1 h, then analyzing the pDNA by agarose gal electrophoresis. Methylated pDNA was protected from BstU I and Hpa II digestion whereas unmethylated or partially methylated pDNA was cleaved. Gel analysis showed that the methylated pDNA was completely protected from either BstU I or Hpa II digestion.

The plasmids used in these studies were highly purified and contained predominantly the supercoiled form, less than 1 endotoxin unit/mg of plasmid and were free of infectious contaminants as determined using a bioburden assay. To assess the role of methylation of CpG dinucleotides in the plasmid DNA on lung inflammation, the purified pDNAs were either methylated or mock methylated *in vitro* using *E. coli* Sss I methylase. This enzyme methylates the cytosine residue (C5) within all CG dinucleotides. The extent of methylation was assessed by monitoring the susceptibility of the modified plasmids to digestion by BstU I or Hpa II but not Msp I. An Sss I-methylated but not the mock-methylated plasmids were completely protected from digestion with BstU I and Hpa II (data not shown). Methylation of pCF1-CAT also resulted in an approximately 5 fold reduction in expression levels following intranasal administration into lungs of BALB/c mice (Figure 7).

Cytokine levels in the mouse BALF were quantitated using enzyme-linked immunosorbent assay (ELISA) kits as specified by the manufacturers. IFN-γ, TNF-α, IL1-α, IL-1β, IL-10 and IL-6 ELISA kits were from Genzyme Corporation, mKC, MIP-2 and GM-CSF ELISA kits were from R&D Systems, and Leukotriene B4 ELISA kit was from Perseptive Diagnostics.

The procedures for processing the lung tissues and assay of CAT enzymatic activity have been described elsewhere. See Lee et al., Hum. Gene Ther., 7, 1701-1717 (1996); Yew et al., Hum. Gene Ther., 8, 575-84 (1997).

### Example 3 Nasal instillation of cationic Iipid:pDNA complexes into mice.

The cationic lipid:pDNA complexes were formed by mixing equal volumes of GL-67:DOPE (1:2) with pDNA as described previously (Lee et al., Hum. Gene Ther., 7, 1701-1717, 1996) to a final concentration of 0.6:1.2:3.6 mM (GL-67:DOPE:pDNA) or 0.3:0.6:1.8 mM, as indicated in the figure legends. The DNA concentration is expressed in terms of nucleotides, using an average nucleotide molecular weight of 330 daltons. BALB/c mice were instilled intranasally with 100 µl of complex as described. See Lee et al., Hum. Gene Ther., 7, 1701-1717 (1996); Scheule et al., Hum. Gene Ther., 8, 689-707 (1997). The animals were euthanized and their lungs were lavaged 24 h post-instillation using phosphate-buffered saline (PBS). The recovered BALF were centrifuged at 1,500 rpm for 4 min, and the resulting supernatants were removed and frozen at -80°C for subsequent cytokine analysis. The cell pellets were resuspended in PBS for microscopic determination of cell number and cell types.

### Example 4 Composition of bronchoalveolar lavage fluid after administration of cationic lipid:pDNA complexes harboring either methylated or unmethylated pDNA.

The Sss I-methylated (m)pDNA or unmethylated pDNA were complexed with the cationic lipid GL-67 and then instilled intranasally into BALB/c mice. Separate groups of mice were instilled with either (m)pDNA or unmethylated pDNA alone, or vehicle, and their bronchoalveolar lavage fluids collected for analysis at 24 h post-treatment.

To determine whether methylation of pDNA affected the inflammatory response in the lungs, we measured the levels of several different cytokines in the BALF 24 h after instillation. Significantly higher levels of TNF-α, IFN-γ, and to a lesser extent IL-6, were found in the BALF of mice that received GL-67:pCF1-CAT when compared to those administered GL-67:(m)pCF1-CAT (Figure 2). Levels of murine KC were also elevated following instillation of the cationic lipid:pDNA complexes but there was no significant difference in the levels of the cytokine induced by either methylated or unmethylated pDNA complexed with GL-67. In contrast, low levels of these four cytokine were present after instillation with GL-67 alone, (m)pCFI-CAT alone or unmethylated pCFI-CAT alone (Figure 2). However, although the levels of TNF-α, IFN-γ and IL-6 were low in the BALF of animals treated with free pDNA compared to complexed pDNA, the levels of these cytokines were invariably higher in the group that received free unmethylated pDNA alone than in the group administered (m)pCF1-CAT. The cytokines IL-10, leukotriene B-4, IL-1β, IL-1α, MIP-2, and GM-CSF were also assayed but in each case the levels were low and indistinguishable from those attained in naive animals (data not shown). These results indicated that unmethylated pDNA was inflammatory in the lung and that this response was exacerbated when the pDNA was present in a complex with GL-67. Furthermore, of the cytokines induced by administration of GL-67:pCF1-CAT complexes to the lung, TNF-α, IFN-γ and a proportion of the IL-6 were primarily due to the presence of unmethylated pDNA. The cationic lipid GL-67 did not contribute significantly to the cytokine induction in the BALF with the exception of KC where it appeared to work in concert with pDNA to increase its level.

The character of the inflammatory response induced by GL-67:pCF1-CAT was also evaluated by measuring the total number of cells and the differential counts recovered in the BALF of the treated animals. Elevated numbers of polymorphonuclear (PMN) leukocytes were present in the BALF of mice that were instilled with GL-67:pDNA compared to mice that received either GL-67 alone or pDNA alone (Figure 3A). The methylation status of the pDNA in the GL-67:pDNA complex did not significantly affect the overall cell number. However, animals administered (m)pCF1-CAT alone (4 separate experiments) consistently showed a slight reduction in the total number of PMN leukocytes in comparison to those that received pCF1-CAT. An analysis of the different cell types showed an increased proportion of neutrophils in mice that received GL-67:pCF1-CAT compared to mice that received GL-67:(m)pCF1-CAT (Figure 3B). This increase was also observed after instillation of pCF1-CAT alone compared to (m)pCF1-CAT alone. Together, these data indicate that the induction in cellular influx was mediated by both the cationic lipid and pDNA. However, administration of unmethylated pDNA rather than methylated pDNA into the lung can result in an increase in the number of PMN leukocytes, particularly neutrophils, in the BALF.

Since pCF1-CAT expresses high levels of the CAT reporter enzyme, which is a bacterial protein, there was the possibility that the cytokine response was due to the expression of the foreign protein. Therefore experiments were repeated using a plasmid vector that contained the same plasmid backbone but lacked any transgene (pCF1-null). The cytokine induction profile after administration of methylated or unmethylated pCF1-null complexed with GL-67 was essentially identical to that attained with pCF1-CAT (data not shown). This confirmed that the plasmid DNA itself and not expression of the bacterial CAT was responsible for the observed cytokine induction.

### Example 5 Dose-dependent relationship between unmethylated pDNA and cytokine levels.

To determine whether there was a dose-dependent relationship between the amount of unmethylated pDNA administered to the lung and the levels of induced cytokines, (m)pCF1-CAT was mixed with pCF1-CAT at different ratios before complexing with GL-67. The dose of GL-67 and the total amount of nucleotides delivered remained constant. In this experiment MIP-2 and IL-12 were assayed in addition to TNF-α, IFN-γ, IL-6, and mKC. As the proportion of unmethylated pCF1-CAT in the complex increased, there was a corresponding increase in the levels of TNF-α, IFN-γ, IL-6, and IL-12 (Figure 4). With IFN-γ, IL-6 and IL-12, the stimulated increase in cytokine levels was maximal when the ratio of methylated:unmethylated pDNA was 1:2. This dose-dependent relationship supports the proposal that the induction of TNF-α, IFN-γ, IL-6, and IL-12 in the BALF were in direct response to the presence of unmethylated pDNA. This trend was not observed for either KC or MIP-2, consistent with the observations above (Figure 4).

### Example 6 Histopathological changes in the lung after administration of cationic lipid:methylated pDNA complexes.

The histopathological changes within BALB/c mouse lungs following administration of either cationic lipid alone, pDNA alone, or cationic lipid:pDNA complexes were also examined. BALB/c mice were instilled intranasally with GL-67:(m)pCF1-CAT, GL-67:pCF1-CAT, GL-67 alone, (m)pCF1-CAT, pCF1-CAT, or water (vehicle control). Mice were sacrificed 2 days post-instillation and the lungs were processed for histological examination in a blinded manner. *Histopathology.*

Lungs were fixed by inflation at 30 cm of H₂O pressure with 2% paraformaldehyde and 0.2% glutaraldehyde. Representative samples were taken from each lung lobe, embedded in glycol methacrylate, sectioned and stained with hematoxylin and eosin. Histopathology on the lung was evaluated in a blinded fashion and graded subjectively using a scale of 0 to 4, where a score of 0 indicates no abnormal findings and a score of 4 reflects severe changes with intense infiltrates See Scheule et al., Hum. Gene Ther., 8, 689-707 (1997).

Multifocal areas of alveolar inflammation were observed in mice that received GL-67:pDNA complexes. The extent of lung inflammation was graded using a scale from 0 to 4, with 0 indicating no abnormalities, 1 indicating a minimal change, 2 a mild change, 3 a moderate change, and 4 representing severe changes from a normal lung (Figure 5). There was no significant difference in the inflammation score of lungs that received GL-67:pDNA compared to lungs that received GL-67:(m)pDNA complex. Lungs that received GL-67 alone were scored slightly lower than lungs that received lipid:pDNA complex, while minimal inflammation was observed in lungs that received either pDNA or (m)pDNA alone. These results indicated that the presence of unmethylated CpG motifs on the pDNA did not grossly affect the histopathological changes observed in the lung after administration of cationic Iipid:pDNA complexes. Furthermore, the majority of the histological changes observed upon administration of the complexes was mediated by the cationic lipid component.

### Example 7 Effect of mutating immunostimulatory CpG motifs within pCF1-CAT

If a subset of the unmethylated CpG dinucleotides present in pCF1-CAT were responsible for the majority of the cytokine response, then elimination of these particular CpG motifs may reduce the level of induction. There are 17 motifs in pCF1-CAT having the sequence 5'-RRCGYY-3, which have been previously shown to be the sequence context in which the CpG motif was found to be most immunostimulatory (Figure 6). Fourteen of these motifs were eliminated by either deletion or site-directed mutagenesis. The four CpG motifs located within the CMV promoter (at nucleotide positions 88, 118, 141 and 224) were removed by deletion of a 400 bp fragment containing a portion of the upstream enhancer region, to create pCFA-299-CAT (Figure 6). Ten of the thirteen remaining motifs (at positions 410, 564, 1497, 1887, 2419, 2600, 2696, 3473, 4394 and 4551) were modified using site-directed mutagenesis to create pCFA-299-10M-CAT (Figure 6). The cytosine residue in each motif was mutated to a thymidine residue in each case, with the exception of one motif (nucleotide 1497) within the coding sequence for CAT, and one motif (nucleotide 4394) within the kanamycin resistance gene. With these two motifs, in order to preserve the coding sequence for the respective proteins, the guanidine residue of the CpG dinucleotide was changed to an adenosine residue. We were unable to mutate the residue at nucleotide 2789 which is located within the proximity of the origin and which we speculate may be essential for plasmid replication.

The plasmids, pCF1-CAT, (m)pCF1-CAT, pCFA-299-CAT, and pCFA-299-10M-CAT were complexed with cationic lipid G L-67 then instilled intranasally into BALB/c mice. Twenty-four hours after instillation, BALF was collected for cytokine analysis and the lungs harvested for CAT assays. Expression from pCFA-299-CAT, containing the truncated CMV promoter, was approximately one-third that of pCF1-CAT (Figure 7). The expression from pCFA-299-10M-CAT was equivalent to pCFA-299-CAT, indicating that the introduction of the 10 point mutations did not affect transgene expression (Figure 7). As before, high levels of TNF-α, IFN-γ, IL-6, and IL-12 were present in the BALF of mice that received unmethylated pCF1-CAT (Figure 8). However, equally high levels of these cytokines were also observed with pCFA-299-CAT and pCFA-299-10M-CAT. Therefore, reducing the content of CpG motifs within the plasmid did not reduce its ability to elevate cytokine levels in the lung. This suggests that removal of other immunostimulatory motifs in addition those harboring the consensus 5'-RRCGYY-3' are necessary to abate the inflammatory response.

### Example 8 Effect of inhibiting influx and cytokine activation in the lung on CAT expression.

Although mutation of the plasmid vector was ineffective at reducing the inflammation in the lung, it has been shown previously that injection of antibodies against Mac-1α and LFA-1 can limit the influx of neutrophils and induction of TNF-α. This method was used to determine the effect of transiently reducing the inflammatory response on CAT expression.

Neutrophil influx into the lungs of BALB/c mice was inhibited by systemic administration of a combination of the antibodies against Mac-1α and LFA-1 as described previously. See Scheule et al., Hum. Gene Ther., 8, 689-707 (1997). The monoclonal antibody recognizing murine Mac-1α (clone M1/70; ATCC; TIB 128) was generated from ascites and that recognizing LFA-1 was obtained from R & D Systems. Briefly, to inhibit neutrophil influx, 100 µl of a mixture containing 40 µl of Mac-1α ascites fluid and 40 µl of anti-LFA-1 antibody were injected by tail vein into the mice at approximately 10 min. prior to administration of the cationic lipid:pDNA complex. Mice were sacrificed at various time points post-instillation, their lungs lavaged, and the resulting BALF analyzed for cytokine levels and cell counts.

Mice were injected via the tail vein with a mixture of the two antibodies just prior to instillation of GL-67:pCF1-CAT into the lung. Cell types and cytokines in the BALF and CAT activity in the lung were assayed at day 2 and 7 post-instillation. In mice that were pretreated with the antibodies, there was a significant decrease in the number of neutrophils; in the BALF as well as decreased levels of TNF-α, IFN-γ, and IL-12 (Figure 9). Concomitant with this decrease in cytokine levels was a greater than 4 fold increase in CAT expression at day 2 post-instillation (Figure 10). Approximately equivalent levels of CAT were present at day 7. These findings indicate, that by reducing the neutrophil influx and cytokine induction in the lung a significant enhancement in transgene expression could be attained.

### Example 9 Effects of modification of the pDNA vector by site-directed metagenesis.

### Mouse spleen cells

6-8 week old 8ALB/c mice were sacrificed by cervical dislocation. Spleens were excised and placed in sterile PBS on ice. To prepare single cell suspensions, the spleens were placed in a tissue culture dish in PBS and crushed between the frosted edges of two sterile microscope slides. Cells were pelleted by centrifugation at 1200 rpm for 8 min., and washed 3 times in PBS. After the final wash, the cells were resuspended in RPMI medium supplemented with 25 mM HEPES buffer, 10% fetal bovine serum, 2 mM L-glutamine, and 50 µM 2-mercaptoethanol (2-ME). The resuspended cells were then plated at 6 x 10⁶ cells /ml/well in 24-well culture plates. Supernatant were collected 24 hr after addition of oligonucleotides or plasmid DNA for the measurement of cytokine (IL-12) production.

### Site-directed mutagenesis

Site-directed mutagenesis was performed using the Quick-change mutagenesis kit (Stratagene) according to the protocol supplied by the manufacturer. In general, the cytosine within the CpG dinucleotide was changed to a adenine. In some cases the guanine was changed so as not to alter the coding sequence. The mutations were confirmed by DNA sequencing.

### Plasmid vector construction

The vector pCFA-CAT has been described previously (see Example 1). A 2.6 kb Sph I fragment from pCFA-CAT containing the kanamycin resistance gene and replication origin region was isolated and ligated to itself to form pOri-K. To construct the CpG reduced plasmids, a 995 bp fragment encoding the 3-aminoglycosidase gene (kanamycin resistance gene) and a 721 bp fragment encoding the E. coil chloramphenicol acetyltransferase (CAT) gene were synthesized by Operon Technologies (Gene0p). A 740 bp fragment encompassing the origin of replication was amplified by the polymerase chain reaction (PCR) from pCFA. This region corresponds to nucleotides 1894 to 2633 of pUC19. The synthetic kanamycin fragment and the origin fragment were ligated to form pOri-K mut. To construct pGZA-CAT, the CAT gene from pCFA-CAT was first replaced with the synthetic CAT gene. From this construct a 2 kb Sph I fragment containing the CMV promoter, intron, synthetic CAT, and bovine growth hormone polyadenylation signal was isolated and ligated to pOri-K-mut to form pGZA-CAT.

### Modification of the pDNA vector by site-directed mutagenesis

The plasmid expression vector pCFA-CAT contains the enhancer and promoter from the immediate early gene of cytomegalovirus, an intron, the E. coli chloramphenicol acetyltransferase reporter gene, the bovine growth hormone polyadenylation signal, a ColE1 replication origin region, and a kanamycin resistance gene. Counting both strands of the plasmid, there are in total 526 CpG dinucleotides (Table 1).

Site-directed mutagenesis was performed on the CpG sites within the replication origin region and kanamycin resistance gene, which contain the largest number of these sites. In most cases the CG dinucleotide was changed to a CA, with some exceptions where the mutations were designed not to alter coding sequence. Within the kanamycin resistance gene thirty two of 117 CpG sites were eliminated. Within the replication origin region many of the attempted, mutations apparently destroyed replication function, and only eight of 161 CpG sites were eliminated. To compare the relative stimulatory activity of the unmodified and mutated replication origin region and kanamycin resistance gene, fragments encompassing these regions were first amplified by the polymerase chain reaction, then equal amounts of each fragment were added to mouse spleen cells and the levels of IL-12 were measured 24 hours later. Both fragments induced high levels of IL-12 (Fig.11), consistent with the known stimulatory activity of E. coll derived DNA. The mutated kanamycin resistance gene induced 50% less IL-12 from the mouse spleen cells compared to the unmodified gene. The mutated replication origin region also induced slightly less IL-12 compared to the unmutated region. These results indicate that reducing the number of CpG sites within a given DNA segment reduces stimulatory activity.

An alternate approach to mutagenesis for reducing the number of CpG sites within the replication origin region was to determine the minimal fragment that could still be functional. Previous studies have shown that the minimal region required for DNA replication encompasses the origin and approximately 999 bp upstream, which encodes the RNA II primer. The region was decreased in size from 1276 bp to 740 bp, eliminated 65 CpG motifs, but remained fully competent for replication. When tested on mouse spleen cells, the shortened replication region induced levels of IL-12 similar to that of the mutated origin. However on a per plasmid basis this minimal replication fragment contributes significantly fewer CpG motifs.

Based on these data, further reductions in stimulatory activity were likely to be achieved by eliminating additional CpG-S sites. Instead of performing additional mutagenesis, the fragment encoding the kanamycin resistance gene was synthesized by assembly of several overlapping oligonucleotides. The sequence was designed to eliminate all CpG dinucleotides without altering the amino acid sequence. The fragment encoding the CAT reporter gene was synthesized in a similar fashion, eliminating 78 of the 60 CpG sites. When tested on mouse spleen cells, these CpG-deficient fragments were essentially non-stimulatory, with levels of IL-12 equal to that of the vehicle control. These results demonstrate that large DNA fragments can be made non-stimulatory by the elimination of CpG sites.

### Example 10 Stimulafor activity of the CpG reduced vector

### Administration of cationic lipid: pDNA complexes into mice.

For systemic delivery, cationic lipid:pDNA complexes were formed by mixing equal volumes of GL-62:DOPE (1:2) with pDNA as described previously (Lee *et al.,* 1996) to a final concentration of 0.6:1.2:3.6 mM (GL-67:DOPE:pDNA). The DNA concentration is expressed in terms of nucleotides, using an average nucleotide molecular weight of 330 Daltons. BALB/c mice were injected via the tail vein with 100 ml of complex. Serum was collected 24 hours post-injection.

For delivery into the lung, complexes of GL-67:DOPE:pDNA were instilled intranasally into BALB/c mice with 100 µl of complex as described (Lee *et al.,* 1996; Scheule *et al.,* 1997). The animals were euthanized and their lungs were lavaged 24 h post-instillation using phosphate-buffered saline (PBS). The recovered BALF were centrifuged at 1,500 rpm for 4 min., and the resulting supernatants were removed and frozen at -80°C for subsequent cytokine analysis.

### Cytokine and CAT activity assays.

Cytokine levels were quantitated using enzyme-linked immunosorbent assay (ELISA) kits as specified by the manufacturer (Genzyme Corporation, Framingham, MA). Our procedures for processing the lung tissues and assay of CAT enzymatic activity have been described elsewhere (Lee *et al.,* 1996; Yew *et al.,* 1997).

### Stimulatory activity of the CpG reduced vector

The synthetic kanamycin resistance gene and the minimal replication origin region were ligated together to form pOri-K-mut. This plasmid contains 96 CpG sites compared to 278 CpG sites in the plasmid pOri-K composed of the unmutated kanamycin resistance gene and unmodified replication origin region. The plasmids were added to mouse spleen cells and the levels of IL-12 in the supernatant were measured 24 hours later. The levels of IL-12 induced by pOri-K-mut were approximately 20% of that induced by pOri-K.

The pOri-K-mut was then used to reassemble the modified form of pCFA-CAT. The CMV enhancer-promoter was left unaltered because of concerns that mutations within this region would decrease promoter activity. The intron/poly A was also unchanged, but was found to be only weakly stimulatory when tested on mouse spleen cells. The synthetic CAT gene was used in place of the unmodified CAT gene. The final reassembled vector pGZA-CAT contains 256 CpG sites compared to 526 sites in pCFA-CAT. When tested on mouse spleen cells the levels of IL-12 were approximately 35% of that induced by pCFA-CAT.

To assess the stimulatory activity of pGZA-CAT *in vivo,* the vector was complexed with cationic lipid GL-62, then injected intravenously into BALB/c mice. Serum was collected 24 hours post injection and the cytokine levels were measured by ELISA. As was shown previously, cationic lipid-pDNA complexes induce high levels of the inflammatory cytokines IFN-γ, IL-12, and IL-6. Compared to the levels induced by GL-62:pCFA-CAT complexes, the levels of IL-12 in the serum after injection of GL-62:pGZA-CAT were decreased 43%, and the levels of IFN-γ and IL-6 were decreased 81% and 78% respectively. The pGZA-CAT vector was also complexed with cationic lipid GL-67, then instilled intranasally into the lungs of BALB/c mice. Bronchoalveolar lavage fluid was collected 24 hours post injection, and the levels of cytokines were measured by ELISA. Compared to the levels induced by GL-67:pCFA-CAT complexes, the levels of IL-12 in the BALF after instillation of GL-67:pGZA-CAT were decreased 55%, and the levels of TNF-a and IL-6 were decreased 55% and 60% respectively.

Here we have eliminated 270 out of 526 CpG dinucleotides in a reporter plasmid (pCFA-CAT) and tested the inflammatory response to cationic lipid:p-DNA complexes containing the modified vector (pGZA-CAT) after systemic or intranasal delivery into BALB/c mice. Compared to the unmodified vector, the CpG-reduced pGZA-CAT was found to be significantly less stimulatory, as the levels of IL-12, IFN-γ, and IL-6 in the serum 24 hours after intravenous delivery were reduced by 40-80%. Similar reductions in cytokine levels were observed in the bronchoalveolar lavage fluids after intranasal administration, while the levels of reporter gene expression were not affected by the modifications.

### Example 11 Inhibition of IL-12 production from mouse spleen cells with chloroquine and quinacrine.

Another strategy to reduce the stimulatory properties of the pDNA vector is to use specific inhibitors of 1 he CpG signaling pathway. Chloroquine and quinacrine have previously been shown to inhibit the immunostimulatory properties of oligonucleotides containing CpG motift *in vitro.* These compounds were tested for their ability to inhibit the stimulatory properties of pDNA and cationic lipid-pDNA complexes *in vitro* and in the mouse lung. pCFA-CAT together with chloroquine or quinacrine was added to mouse spleen cells and the levels of IL-12 in the culture medium were measured 24 hours later. 10 µM of chloroquine or 1µM of quinacrine effectively decreased the levels of IL-12 induction to near background levels (Fig.15). To determine if chloroquine and quinacrine could inhibit stimulation by cationic lipid-pDNA complexes, chloroquine and quinacrine were added together With a complex of cationic lipid GL-67 and pCFA-CAT. 10 µM of chloroquine or 1 µM of quinacrine again decreased the levels of IL-12 induction to near background levels (Fig. 15).

### Exampe 12 Cytokine profiles in bronchoalveolar lavage fluid after instillation of cationic lipid:pDNA complex plus chloroquine or quinacrine.

Chloroquine and quinacrine were also instilled with complexes of GL-67 and pCFA-CAT into the lungs of BALB/c mice. BALF was collected 24 hours post-instillation and cytokines were measured by ELISA. The addition of 0.1 µM chloroquine or 0.1 µM quinacrine decreased the levels of IL-12, TNF-α, and IFN-γ by 50 to 70% compared to levels after instillation of complex alone. Higher concentrations of either chloroquine or quinacrine did not further decrease cytokine levels (data not shown). The levels of CAT expression were not affected by the addition of either compound (Fig. 16). These results suggest that low concentrations of chloroquine and quinacrine can effectively reduce the inflammatory response associated with instillation of cationic lipid-pDNA complexes into the lung.

The results demonstrate that known inhibitors of the CpG signaling pathway reduce the levels of inflammatory cytokines. Two such inhibitors, chloroquine and quinacrine, greatly reduced the induction of IL-12 production from mouse spleen cells *in vitro,* and inhibited -cytokine production in the lung by 50% without affecting gene expression. The use of a less stimulatory pDNA vector together with inhibitors of CpG stimulation reduce the toxicity associated with cationic lipid-pDNA complexes and thereby increase the safety of non-viral gene therapy.

### Example 13 The role of the methylation state of the CpG motifs in pCF1CAT on the toxicity observed following systemic administration of GL-67:pCF1CAT complex.

GL-67:DOPE:DMPEPeg5000 (1:2:0.05; molar ratio) was hydrated in sterile, pyrogen-free water to a concentration of 4 mM GL-67. Plasmid DNA(pDNA) was diluted in sterile, pyrogen-free water to a concentration of 4 mM. GL-67:pDNA complex was prepared by adding an equal volume of cationic lipid suspension to an equal volume of pDNA followed by gentle mixing to achieve homogeneity of the suspension. The mixture was then incubated for a minimum of 15 minutes and a maximum of 60 minutes before injection. Complex was prepared using the following types of pDNA: 1) untreated pCF1CAT [pCF1CAT which has not been modified following column purification ]2) methylated pCF1CAT [pCF1CAT on which the CpG motifs have been methylated with *SSS-1* methylase, 3) mock methylated pCF1CAT [pCF1CAT which has undergone the methylation reaction but where the methylase was absent from the reaction mixture].

Eight female BALB/c mice per group were injected with a 100 µl bolus of either GL-67:pDNA complex or vehicle via the tail vein. At approximately 24 hours post-injection, whole blood and serum were collected from the mice by retro-orbital bleed. Whole blood underwent a complete hematology scan for which a representative but not exclusive set of parameters follows: white blood cell count (WBC), white blood cell differential, red blood cell count (RBC), hemoglobin, and platelet count. Serum was also analyzed for a small animal serum chemistry profile for which a representative but not exclusive set of parameters follows; serum transaminases (alanine aminotransferase [ALT], aspartate aminotransferase [AST]), creatinine kinase, bilirubin, serum protein levels including albumin and globulin levels, blood urea nitrogen, electrolytes, and glucose. Serum was also tested using enzyme-linked immunoassay (ELISA) kits from R&D Systems for the presence of the following representative cytokines-interleukin 6 (IL-6), interleukin-12 (IL-12), and interferon-gamma (IFN-γ).

Prior toxicology studies have indicated that the physiological changes mediated by systemic administration of cationic lipid:pDNA complex are characterized in part by statistically significant elevations in serum transaminase (ALT, AST) levels and in the cytokines IL- 6, IL-12, and IFN-γ as compared to vehicle treated animals. Elevations in serum transaminase (ALT, AST) levels are generally accepted as indicators of liver toxicity, although other clinical measures of liver damage are certainly recognized and could be substituted. Two sources for more detailed information on the diagnostic tests and uses of clinical chemistry are Fundamentals of Clinical Chemistry, N.W. Tietz, Editor, Saunders, Philadelphia, 1976 and Clinical Diagnosis by Laboratory Methods, 15th ed., Saunders, Philadelphia, 1974. Cytokine levels were measured as an indicator of inflammation; the panel of cytokines selected are generally accepted as a pro-inflammatory set. However, prior toxicology studies indicate that induction of more than this subset of cytokines occurs following systemic administration of cationic lipid:pDNA complex. Therefore, this cytokine panel should be viewed as a representative but not exclusive measurement of the cytokine response generated by cationic Iipid:pDNA complex. A source of additional information on the relation of inflammation and cytokines/ other blood soluble mediators of cell to cell interactions can be found in Immunology, 5th ed., Mosby International Ltd, London, 1998. *Results*

Mice injected with both GL-67:untreated pCF1CAT complex and GL-67:mock methylated pCF1CAT complex show significant elevations in serum transaminase (ALT, AST) levels as well as a significant elevations in levels of IL-6, IL-12, and IFN-γ as compared to vehicle treated animals. Mice injected with GL-67:methylated pCF1CAT complex also show significant elevations in serum transaminase (ALT, AST) levels but have levels of IL-12 and IFN-γ which are close to those observed in vehicle treated animals. These results illustrate that methylation of CpG motifs in pCF1CAT blocks induction of IL-12 and IFN-γ expression but does not alter the systemic toxicity observed following administration of GL-67:pCF1 CAT complex as indicated by serum transaminase levels.

### Example 14 The role of CpG motifs in pCF1CAT on the toxicity observed following systemic administration of GL-67:pCF1CAT complex.

GL-67:DOPE:DMPEPeg5000 (1:2:0.05; molar ratio) was hydrated in sterile, pyrogen-free water to a concentration of 4 mM GL-67. Plasmid DNA(pDNA) was diluted in sterile, pyrogen-free water to a concentration of 4 mM. GL-67:pDNA complex was prepared by adding an equal volume of cationic lipid suspension to an equal volume of pDNA followed by gentle mixing to achieve homogeneity of the suspension. The mixture was then incubated for a minimum of 15 minutes and a maximum of 60 minutes before injection. Complex was prepared using the following types of pDNA: 1) pCF1CAT and 2) pGZACAT (a plasmid vector in which the total number of CpG motifs has been reduced by 50% relative to pCF1CAT).

Eight female BALB/c mice per group were injected with a 100 µl bolus of either GL-67:pDNA complex or vehicle via the tail vein. At approximately 24 hours post-injection, whole blood and serum were collected from the mice by retro-orbital bleed. Whole blood was sent for a complete hematology scan for which a representative but not exclusive set of parameters follows: white blood cell count (WBC), white blood cell differential, red blood cell count (RBC), hemoglobin, and platelet count. Serum was also sent for a small animal serum chemistry profile for which a representative but not exclusive set of parameters follows; serum transaminases (alanine aminotransferase [ALT], aspartate aminotransferase [AST]), creatinine kinase, bilirubin, serum protein levels including albumin and globulin levels, blood urea nitrogen, electrolytes, and glucose. Serum was also tested using enzyme-linked immunoassay (ELISA) kits from R&D Systems for the presence of the following representative cytokines-interleukin 6 (IL-6), interleukin-12 (IL-12), and interferon-gamma (IFN-γ).

Prior toxicology studies have indicated that the physiological changes mediated by systemic administration of cationic lipid:pDNA complex are characterized in part by statistically significant elevations in serum transaminase (ALT, AST) levels and in the cytokines IL- 6, IL-12, and IFN-γ as compared to vehicle treated animals. Elevations in serum transaminase (ALT, AST) levels are generally accepted as indicators of liver toxicity, although other clinical measures of liver damage are certainly recognized and could be substituted. Two sources for more detailed information on the diagnostic tests and uses of clinical chemistry are Fundamentals of Clinical Chemistry, N.W. Tietz, Editor, Saunders, Philadelphia, 1976 and Clinical Diagnosis by Laboratory Methods, 15th ed., Saunders, Philadelphia, 1974. Cytokine levels were measured as an indicator of inflammation; the panel of cytokines selected are generally accepted as a pro-inflammatory set. However, prior toxicology studies indicate that induction of more than this subset of cytokines occurs following systemic administration of cationic lipid:pDNA complex. Therefore, this cytokine panel should be viewed as a representative but not exclusive measurement of the cytokine response generated by cationic lipid:pDNA complex. A source of additional information on the relation of inflammation and cytokines/ other blood soluble mediators of cell to cell interactions can be found in Immunology, 5th ed., Mosby International Ltd, London, 1998.

### Results

Mice injected with GL-67:pCF1CAT complex show significant elevations in serum transaminase (ALT, AST) levels as well as a significant elevations of IL-6, IL-12, and IFN-γ as compared to vehicle treated animals. In contrast, mice injected with GL-67 complex prepared with pGZACAT (a plasmid vector in which the total number of CpG motifs has been reduced by 50% relative to pCF1CAT) show serum transaminase (ALT ,AST) levels close to those found in vehicle treated animals. These latter animals also show much lower serum levels of IL-6, IL-12, and IFN-γ than the animals treated with GL-67:pCF1 CAT complex where the plasmid is unmodified with respect to CpG motifs. These results illustrate that eliminating CpG motifs from the plasmid vector reduces both the inflammatory response and the systemic toxicity observed following systemic delivery of GL-67:pDNA complex as indicated by the cytokine profile and serum transaminase levels, respectively.

## Claims

1. Use of a plasmid comprising a gene of interest for the manufacture of a medicament for the treatment of a mammal by gene therapy, wherein the use comprises modifying the plasmid by removing or mutating to a non-CpG dinucleotide at least one CpG dinucleotide within said plasmid, and wherein the treatment comprises administering the plasmid in combination with a cationic amphiphile.

2. The use according to Claim 1, wherein the use comprises removing or mutating to a non-CpG dinucleotide at least one CpG dinucleotide within the gene of interest.

3. The use according to Claim 1 or Claim 2, wherein the plasmid comprises an origin of replication, and the use comprises removing or mutating to a non-CpG dinucleotide at least one CpG dinucleotide within the origin of replication.

4. The use according to any one of Claims 1, 2 or 3, wherein the plasmid comprises a polynucleotide encoding a selectable marker, and the use comprises removing or mutating to a non-CpG dinucleotide at least one CpG dinucleotide within said polynucleotide.

5. The use according to any preceding claim, wherein the transgene encodes the cystic fibrosis (CF) transmembrane conductance regulator protein.

6. The use according to any preceding claim, wherein the medicament comprises a pharmaceutical excipient.

7. The use according to Claim 6, wherein the use comprises lyophilizing the plasmid in the presence of said excipient.

8. The use according to any preceding claim, wherein the medicament comprises the cationic amphiphile that is to be administered in combination with the plasmid.

9. The use according to any preceding claim, wherein the cationic amphiphile is a cationic lipid.

10. The use according to any preceding claim, wherein the plasmid and the cationic amphiphile are to be administered together as an aerosolized solution.

11. A process of preparing a pharmaceutical composition for use in gene therapy in a mammal, wherein the pharmaceutical composition comprises: (i) a plasmid comprising a gene of interest; and (ii) a cationic amphiphile; and wherein the process comprises: (a) modifying the plasmid by removing or mutating to a non-CpG dinucleotide at least one CpG dinucleotide within the plasmid; and (b) combining the plasmid with the cationic amphiphile.

## Patentansprüche

1. Verwendung eines ein interessierendes Gen umfassenden Plasmids zur Herstellung eines Arzneimittels für die Behandlung eines Säugers mittels Gentherapie, wobei die Verwendung das Modifizieren des Plasmids durch Entfernen wenigstens eines in dem Plasmid vorliegenden CpG-Dinukleotids oder durch dessen Mutation zu einem Nicht-CpG-Dinukleotid umfasst und wobei die Behandlung das Verabreichen des Plasmids in Kombination mit einem kationischen Amphiphil umfasst.

2. Verwendung nach Anspruch 1, wobei die Verwendung das Entfernen wenigstens eines in dem interessierenden Gen vorliegenden CpG-Dinukleotids oder dessen Mutation zu einem Nicht-CpG-Dinukleotid umfasst.

3. Verwendung nach Anspruch 1 oder Anspruch 2, wobei das Plasmid einen Replikationsursprung umfasst und die Verwendung das Entfernen wenigstens eines in dem Replikationsursprung vorliegenden CpG-Dinukleotids oder dessen Mutation zu einem Nicht-CpG-Dinukleotid umfasst.

4. Verwendung nach einem der Ansprüche 1, 2 oder 3, wobei das Plasmid ein für einen selektionierbaren Marker codierendes Polynukleotid umfasst und die Verwendung das Entfernen wenigstens eines in dem Polynukleotid vorliegenden CpG-Dinukleotids oder dessen Mutation zu einem Nicht-CpG-Dinukleotid umfasst.

5. Verwendung nach einem vorhergehenden Anspruch, wobei das Transgen für das CFTR(Cystic Fibrosis Transmembrane Conductance Regulator)-Protein codiert.

6. Verwendung nach einem vorhergehenden Anspruch, wobei das Arzneimittel einen pharmazeutischen Hilfsstoff umfasst.

7. Verwendung nach Anspruch 6, wobei die Verwendung das Lyophilisieren des Plasmids in Gegenwart des Hilfsstoffs umfasst.

8. Verwendung nach einem vorhergehenden Anspruch, wobei das Arzneimittel das in Kombination mit dem Plasmid zu verabreichende kationische Amphiphil umfasst.

9. Verwendung nach einem vorhergehenden Anspruch, wobei es sich bei dem kationischen Amphiphil um ein kationisches Lipid handelt.

10. Verwendung nach einem vorhergehenden Anspruch, wobei das Plasmid und das kationische Amphiphil zusammen in Form einer aerosolisierten Lösung zu verabreichen sind.

11. Verfahren zur Herstellung einer pharmazeutischen Zusammensetzung für die gentherapeutische Verwendung bei einem Säuger, wobei die pharmazeutische Zusammensetzung (i) ein ein interessierendes Gen umfassendes Plasmid und (ii) ein kationisches Amphiphil umfasst; und wobei das Verfahren (a) das Modifizieren des Plasmids durch Entfernen wenigstens eines in dem Plasmid vorliegenden CpG-Dinukleotids oder durch dessen Mutation zu einem Nicht-CpG-Dinukleotid und (b) das Kombinieren des Plasmids mit dem kationischen Amphiphil umfasst.

## Revendications

1. Utilisation d'un plasmide comprenant un gène d'intérêt, pour la fabrication d'un médicament destiné au traitement d'un mammifère par thérapie génique, l'utilisation comprenant la modification du plasmide par élimination ou mutation en un dinucléotide non-CpG d'au moins un dinucléotide CpG présent dans ledit plasmide, et le traitement comprenant l'administration du plasmide en association avec un composé amphiphile cationique.

2. Utilisation selon la revendication 1, l'utilisation comprenant l'élimination ou la mutation en un dinucléotide non-CpG d'au moins un dinucléotide CpG présent dans le gène d'intérêt.

3. Utilisation selon la revendication 1 ou la revendication 2, dans laquelle le plasmide comprend une origine de réplication, et l'utilisation comprend l'élimination ou la mutation en un dinucléotide non-CpG d'au moins un dinucléotide CpG présent dans l'origine de réplication.

4. Utilisation selon l'une quelconque des revendications 1, 2 ou 3, dans laquelle le plasmide comprend un polynucléotide codant un marqueur sélectionnable, et l'utilisation comprend l'élimination ou la mutation en un dinucléotide non-CpG d'au moins un dinucléotide CpG présent dans ledit polynucléotide.

5. Utilisation selon l'une quelconque des revendications précédentes, le transgène codant la protéine régulatrice de la conductance transmembranaire de la fibrose kystique (CF).

6. Utilisation selon l'une quelconque des revendications précédentes, dans laquelle le médicament comprend un excipient pharmaceutique.

7. Utilisation selon la revendication 6, l'utilisation comprenant la lyophilisation du plasmide en présence dudit excipient.

8. Utilisation selon l'une quelconque des revendications précédentes, dans laquelle le médicament comprend le composé amphiphile cationique qui est destiné à être administré en association avec le plasmide.

9. Utilisation selon l'une quelconque des revendications précédentes, dans laquelle le composé amphiphile cationique est un lipide cationique.

10. Utilisation selon l'une quelconque des revendications précédentes, dans laquelle le plasmide et le composé amphiphile cationique sont destinés à être administrés ensemble sous forme d'une solution aérosolisée.

11. Procédé de préparation d'une composition pharmaceutique pour utilisation en thérapie génique chez un mammifère, dans lequel la composition pharmaceutique comprend : (i) un plasmide comprenant un gène d'intérêt ; et (ii) un composé amphiphile cationique ; et le procédé comprenant : (a) la modification du plasmide par élimination ou mutation en un dinucléotide non-CpG d'au moins un dinucléotide CpG présent dans le plasmide ; et (b) l'association du plasmide avec le composé amphiphile cationique.
